# EUROPEAN PATENT APPLICATION

(11) **EP 4 763 191 A1**
(43) Date of publication of application: **24.06.2026**
(21) Application number: 24853883.7
(22) Date of filing: 16.08.2024
(51) Int. Cl.: A61K 9/00, A61K 9/20, A61K 9/28, A61K 9/48, A61K 9/16, A61K 31/4985, A61K 31/506, C07D 487/04, A61P 35/00

(54) **PHARMACEUTICAL PREPARATION OF PIPERAZINE DERIVATIVE, PREPARATION METHOD THEREFOR, AND USE THEREOF**

(30) Priority: 17.08.2023 CN 202311038440; 28.09.2023 CN 202311282313
(71) Applicant: Kangbaida (Sichuan) Biotechnology Co., Ltd., Chengdu, Sichuan 610000 (CN)
(72) Inventor: CHU, Hongzhu, Chengdu, Sichuan 610000 (CN); LIU, Zhaojun, Chengdu, Sichuan 610000 (CN); SU, Huiqin, Chengdu, Sichuan 610000 (CN); ZHU, Yuqin, Chengdu, Sichuan 610000 (CN); WEI, Yonggang, Chengdu, Sichuan 610000 (CN)
(74) Representative: Dehns
(86) International application number: PCT/CN2024/112655
(87) International publication number: WO 2025/036478

(57) **Abstract**

The present invention relates to a pharmaceutical preparation containing a piperazine derivative, or a stereoisomer, tautomer, deuterated compound or pharmaceutically acceptable salt thereof, a preparation method for the pharmaceutical preparation, and use thereof in medicine. The invention particularly relates to a preparation containing a compound represented by formula (I).

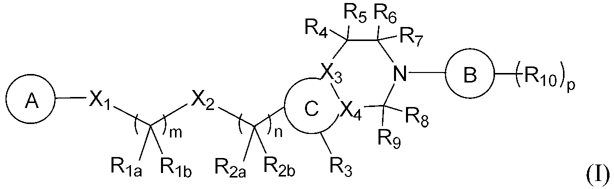

## Description

### Technical Field

The present invention relates to a pharmaceutical preparation comprising a piperazine derivative, or a stereoisomer, tautomer, deuterated compound or pharmaceutically acceptable salt thereof, a preparation method therefor, and the use thereof in medicine.

### Background Art

Adenosine diphosphate-ribosylation (ADP-ribosylation) is a post-translational protein modification process in which single or multiple adenosine diphosphate ribose (ADP-ribose) moieties are added onto amino acid residues of a protein. ADP-ribosylation is a reversible process involved in physiological regulation such as cell signal transduction, DNA damage repair, transcription, gene expression regulation and apoptosis. The enzyme that mediates the addition of ADP-ribose for modification is called ADP-ribosyltransferase. The ADP-ribosyltransferase can catalyse two types of modification: mono-ADP-ribosylation and poly-ADP-ribosylation. When DNA is damaged or cells are stressed, PARP (poly ADP-ribose polymerase) will be activated, resulting in an increase in the amount of poly ADP-ribose and a decrease in the amount of NAD+. To date, scientists have identified 17 different PARPs. MonoPARPs constitute the majority of the PARP family and mediate important biological functions and various stress responses, such as the unfolded protein response, NF-κB signalling, antiviral responses, and cytokine signalling. 2,3,7,8-Tetrachlorodibenzo-p-dioxin (TCDD)-inducible poly(ADP-ribose) polymerase (PARP-7) is one of the members of the MonoPARP family whose expression is regulated by the TCDD-activated aryl hydrocarbon receptor (AHR). AHR is a ligand-activated transcription factor, which can mediate the toxic activity of many environmental xenobiotics. AHR upregulates the expression of PARP-7, which will interact with the kinase TBK1 and cause the ADP-ribosylation of the kinase TBK1, thus inhibiting the TBK1 activity, downregulating the IFN-I (type I interferon) response, and ultimately suppressing the body's antiviral and antitumour immune responses.

Patent (PCT Publication No.: WO 2022242750) describes a novel PARP7 inhibitor represented by formula (I), which has a good inhibitory effect on PARP7 activity and shows the potential to prepare anti-tumour drugs.

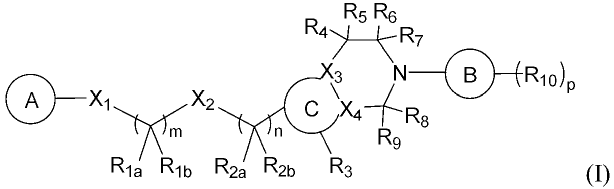

It is essential to prepare a pharmaceutical preparation containing a PARP7 inhibitor that exhibits a good drug dissolution effect and stability.

### Summary

An object of the present invention is to provide a pharmaceutical preparation comprising a PARP7 inhibitor, a preparation method therefor and the use thereof in the preparation of an anti-tumour drug, wherein the PARP7 inhibitor is a piperazine derivative, or a stereoisomer, tautomer, deuterated compound or pharmaceutically acceptable salt thereof.

One or more embodiments of the present invention provide a pharmaceutical preparation of a piperazine derivative, comprising a piperazine derivative, or a stereoisomer, tautomer, deuterated compound or pharmaceutically acceptable salt thereof, wherein the pharmaceutical preparation comprises the piperazine derivative in a strength of 1-1000 mg, and the piperazine derivative is selected from a compound of formula (I): 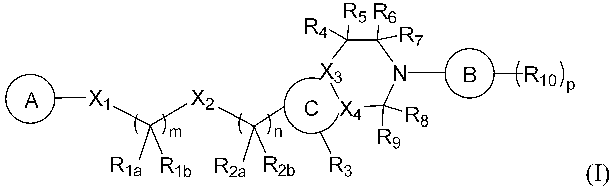 wherein:
X₁ is NH, O or 4- to 6-membered heterocycle containing 1 to 3 heteroatoms selected from N and O;
X₂ is O or a bond;
X₃ and X₄ are each independently C or N;
R₁ₐ and R_{1b} are each independently H, D or C₁₋₆ alkyl; or R₁ₐ and R_{1b} together with the carbon atom connected thereto form 3- to 5-membered cycloalkyl;
R₂ₐ and R_{2b} are each independently H, D or C₁₋₆ alkyl; or R₂ₐ and R_{2b} together with the carbon atom connected thereto form 3- to 5-membered cycloalkyl;
R₃ is H, D, C₁₋₆ alkyl, halogen or cyano, wherein the C₁₋₆ alkyl is optionally substituted with 1 to 3 halogens;
R₄ and R₅ are each independently H, D or C₁₋₆ alkyl; or R₄ and R₅ together with the carbon atom connected thereto form 3- to 5-membered cycloalkyl;
R₆ and R₇ are each independently H, D or C₁₋₆ alkyl; or R₆ and R₇ together with the carbon atom connected thereto form =O;
R₈ and R₉ are each independently H, D or C₁₋₆ alkyl; or R₈ and R₉ together with the carbon atom connected thereto form =O; or R₈ and R₉ together with the carbon atom connected thereto form 3- to 5-membered cycloalkyl;
each R₁₀ is independently C₁₋₆ alkyl, C₁₋₆ alkoxy, CONR₁₀ₐR_{10b}, halogen, cyano, S(O)₂R_{10c}, SR_{10d} or 3- to 5-membered cycloalkyl, wherein the C₁₋₆ alkyl and C₁₋₆ alkoxy are optionally substituted with 1 to 3 halogens;
R₁₀ₐ, R_{10b}, R_{10c} and R_{10d} are each independently H, D or C₁₋₆ alkyl;
A is 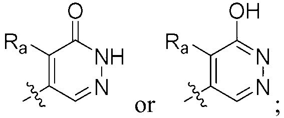
Rₐ is C₁₋₆ alkyl, C₃₋₅ cycloalkyl, halogen or cyano, wherein the C₁₋₆ alkyl is optionally substituted with 1 to 3 halogens;
B is 5- to 10-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3;
n is 0, 1, 2 or 3; and
p is 0, 1, 2 or 3.

In one or more embodiments, the pharmaceutical preparation of the present invention is characterized in that
X₁ is NH, O or 4- to 6-membered heterocycle containing 1 to 3 heteroatoms selected from N and O;
X₂ is O or a bond;
X₃ and X₄ are each independently C or N;
R₁ₐ and R_{1b} are each independently H, D or C₁₋₆ alkyl;
R₂ₐ and R_{2b} are each independently H, D or C₁₋₆ alkyl; or R₂ₐ and R_{2b} together with the carbon atom connected thereto form 3- to 5-membered cycloalkyl;
R₃ is H, D, C₁₋₆ alkyl, halogen or cyano, wherein the C₁₋₆ alkyl is optionally substituted with 1 to 3 halogens;
R₄ and R₅ are each independently H, D or C₁₋₆ alkyl; or R₄ and R₅ together with the carbon atom connected thereto form 3- to 5-membered cycloalkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form =O;
each R₁₀ is independently C₁₋₆ alkyl, C₁₋₆ alkoxy, CONR₁₀ₐR_{10b}, halogen, cyano, S(O)₂R_{10c}, SR_{10d} or 3- to 5-membered cycloalkyl, wherein the C₁₋₆ alkyl and C₁₋₆ alkoxy are optionally substituted with 1 to 3 halogens;
R₁₀ₐ, R_{10b}, R_{10c} and R_{10d} are each independently H, D or C₁₋₆ alkyl;
A is 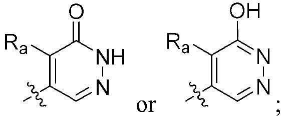
Rₐ is C₁₋₆ alkyl, C₃₋₅ cycloalkyl, halogen or cyano, wherein the C₁₋₆ alkyl is optionally substituted with 1 to 3 halogens;
B is 5- to 6-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3;
n is 0, 1, 2 or 3; and
p is 0, 1, 2 or 3.

In one or more embodiments, the pharmaceutical preparation of the present invention is characterized in that the piperazine derivative is selected from a compound of formula (1-1): 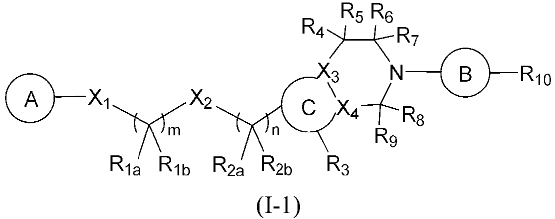 wherein:
X₁ is NH or 4- to 6-membered heterocycle containing 1 to 3 heteroatoms selected from N and O;
X₂ is O;
X₃ and X₄ are each independently C or N;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form =O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 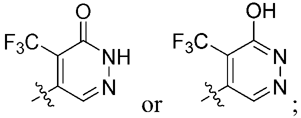
B is 5- to 6-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3; and
n is 0, 1, 2 or 3.

In one or more embodiments, the pharmaceutical preparation of the present invention is characterized in that the piperazine derivative is selected from a compound of formula (I-2): 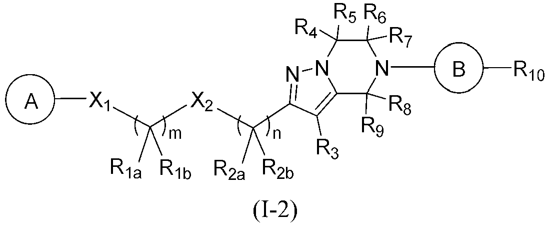 wherein:
X₁ is NH;
X₂ is O;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form =O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 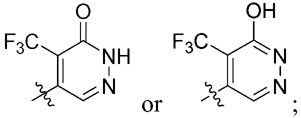
B is 5- to 6-membered carbocycle or heterocycle, wherein the heterocycle contains 1 to 3 heteroatoms selected from N, O and S;
m is 1, 2 or 3; and
n is 0, 1, 2 or 3.

In one or more embodiments, the pharmaceutical preparation of the present invention is characterized in that
X₁ is NH;
X₂ is O;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form =O;
R₁₀ is C₁₋₆ alkyl, cyano or SR_{10d}, wherein the C₁₋₆ alkyl is optionally substituted with 1 to 3 halogens;
R_{10d} is H, D or C₁₋₆ alkyl;
A is 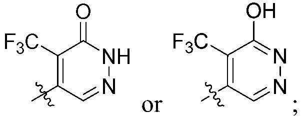
B is 5- to 6-membered carbocycle or heterocycle, wherein the heterocycle contains 1 to 3 heteroatoms selected from N, O and S;
m is 1, 2 or 3; and
n is 0, 1, 2 or 3.

In one or more embodiments, the pharmaceutical preparation of the present invention is characterized in that
X₁ is selected from NH;
X₂ is selected from O;
R₁ₐ and R_{1b} are each independently selected from H, D or C₁₋₆ alkyl;
R₂ₐ and R_{2b} are each independently selected from H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H or D;
R₆ and R₇ are each independently H or D;
R₈ and R₉ are each independently H or D;
R₁₀ is CF₃ or SR_{10d};
R_{10d} is H, D or C₁₋₆ alkyl;
A is 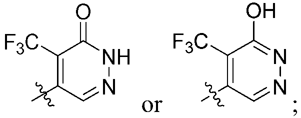
B is 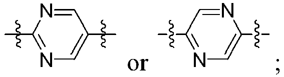
m is 1, 2 or 3; and
n is 0, 1, 2 or 3.

In one or more embodiments, the pharmaceutical preparation of the present invention is characterized in that
X₁ is selected from NH;
X₂ is selected from O;
R₁ₐ and R_{1b} are each independently selected from H, D or C₁₋₃ alkyl;
R₂ₐ and R_{2b} are each independently selected from H, D or C₁₋₃ alkyl;
R₃ is selected from H, D or CF₃;
R₄ and R₅ are each independently selected from H or D;
R₆ and R₇ are each independently selected from H or D;
R₈ and R₉ are each independently selected from H or D;
R₁₀ is CF₃;
A is 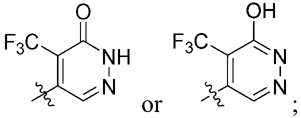
B is 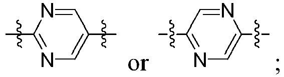
m is 1, 2 or 3; and
n is 0, 1 or 2.

In one or more embodiments, the pharmaceutical preparation of the present invention is characterized in that the piperazine derivative is selected from a compound of formula (I-3): 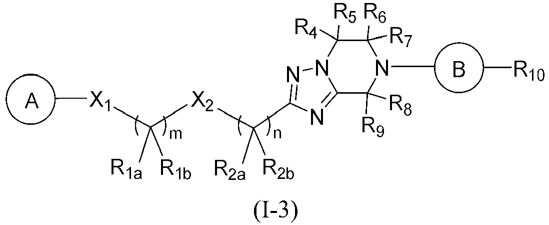 wherein:
X₁ is NH or 4- to 6-membered heterocycle containing 1 to 3 heteroatoms selected from N and O;
X₂ is O;
R₁ₐ and R_{1b} are each independently H, D or C₁₋₆ alkyl;
R₂ₐ and R_{2b} are each independently H, D or C₁₋₆ alkyl;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form =O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 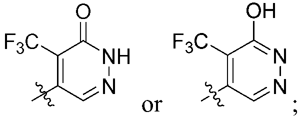
B is 5- to 6-membered carbocycle or heterocycle, wherein the heterocycle contains 1 to 3 heteroatoms selected from N, O and S;
m is 1, 2 or 3; and
n is 0, 1, 2 or 3.

In one or more embodiments, the pharmaceutical preparation of the present invention is characterized in that
X₁ is NH or 4- to 6-membered heterocycle containing 1 to 3 heteroatoms selected from N and O;
X₂ is O;
R₁ₐ and R_{1b} are each independently H, D or C₁₋₃ alkyl;
R₂ₐ and R_{2b} are each independently H, D or C₁₋₃ alkyl;
R₄ and R₅ are each independently H, D or C₁₋₃ alkyl;
R₆ and R₇ are each independently H, D or C₁₋₃ alkyl;
R₈ and R₉ are each independently H, D or C₁₋₃ alkyl; or R₈ and R₉ together with the carbon atom connected thereto form =O;
R₁₀ is C₁₋₆ alkyl, cyano or SR_{10d}, wherein the C₁₋₆ alkyl is optionally substituted with 1 to 3 halogens;
R_{10d} is H, D or C₁₋₆ alkyl;
A is 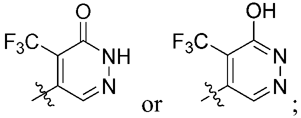
B is 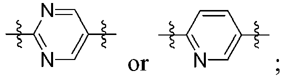
m is 1, 2 or 3; and
n is 0, 1 or 2.

In one or more embodiments, the pharmaceutical preparation of the present invention is characterized in that
X₁ is NH;
X₂ is O;
R₁ₐ and R_{1b} are each independently H, D or C₁₋₃ alkyl;
R₂ₐ and R_{2b} are each independently H or D;
R₄ and R₅ are each independently H or D;
R₆ and R₇ are each independently H or D;
R₈ and R₉ are each independently H or D;
R₁₀ is CF₃;
A is 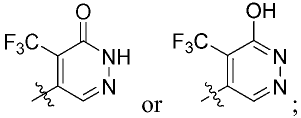
B is 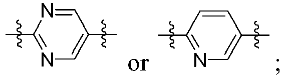
m is 1, 2 or 3; and
n is 0, 1 or 2.

In one or more embodiments, the pharmaceutical preparation of the present invention is characterized in that the piperazine derivative, or the stereoisomer, tautomer, deuterated compound or pharmaceutically acceptable salt thereof is selected from: 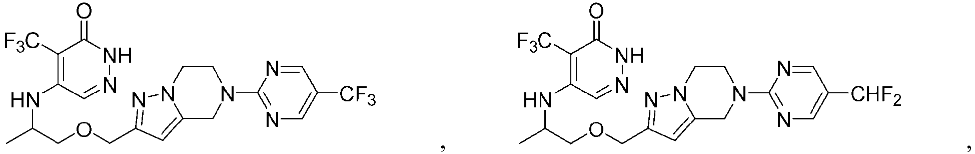 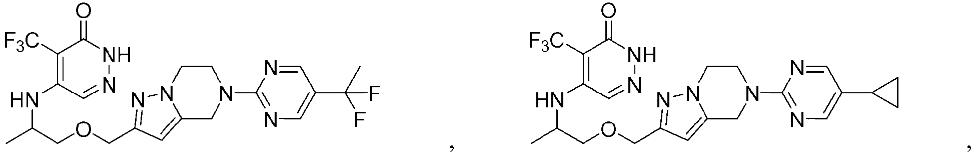 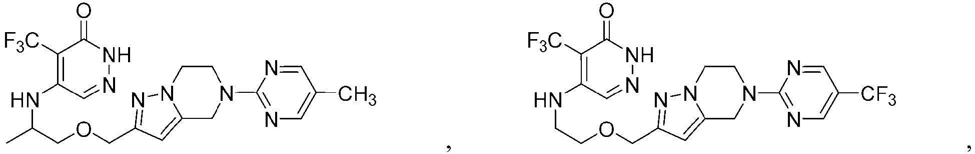 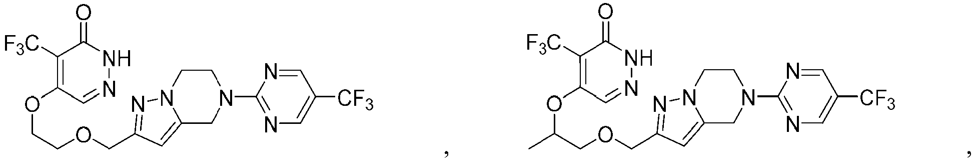 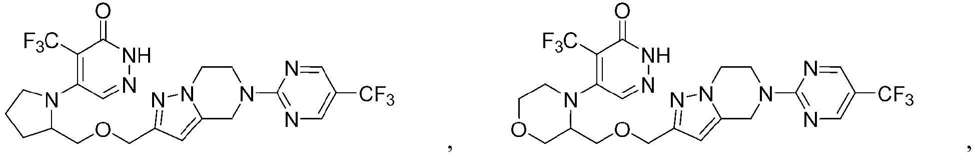 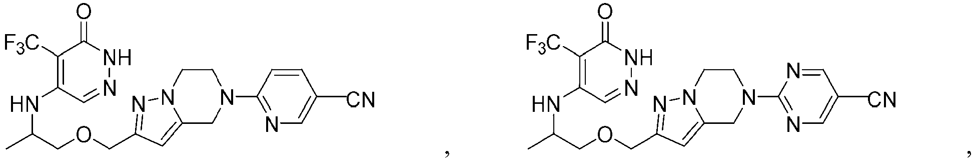 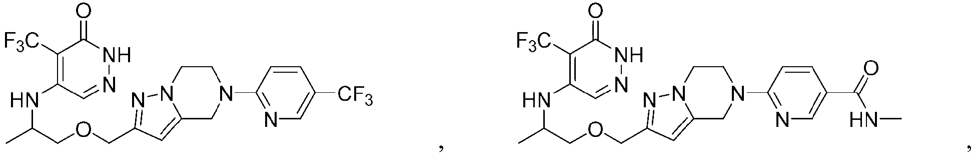 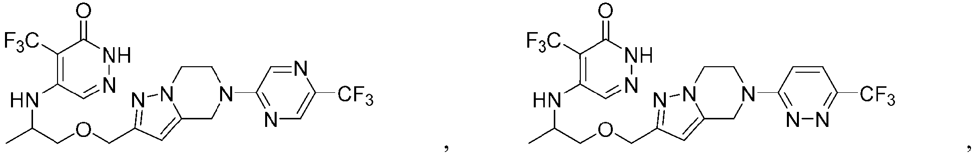 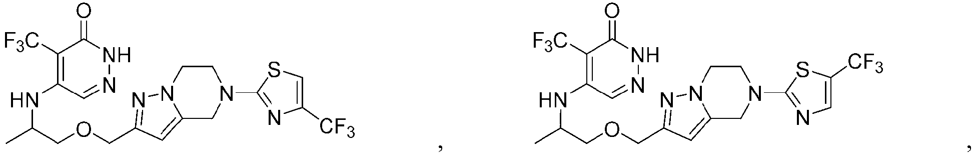 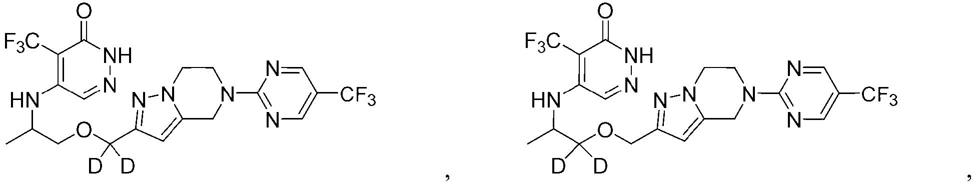 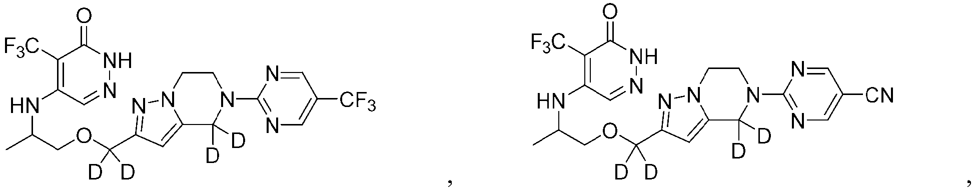 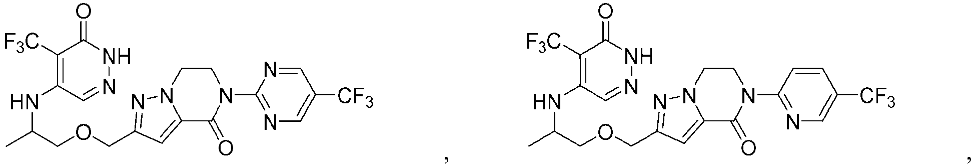 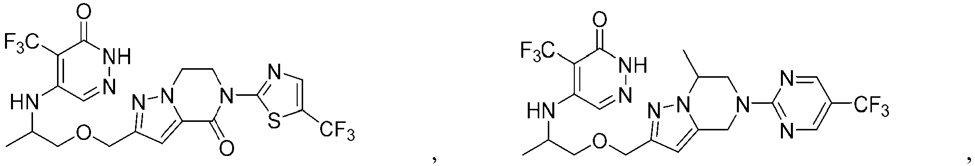 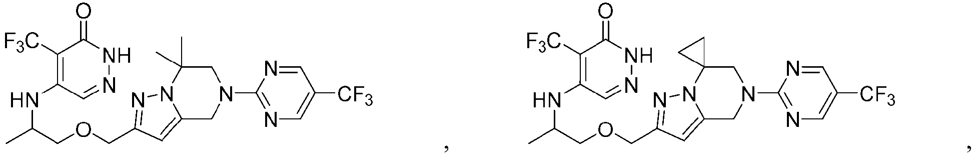 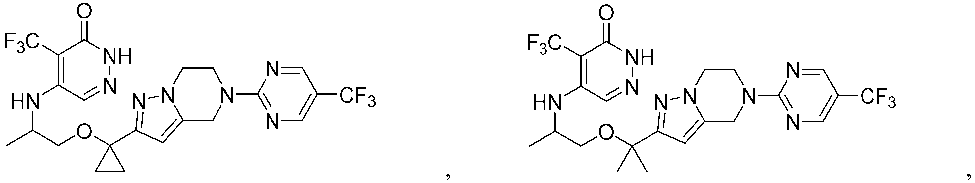 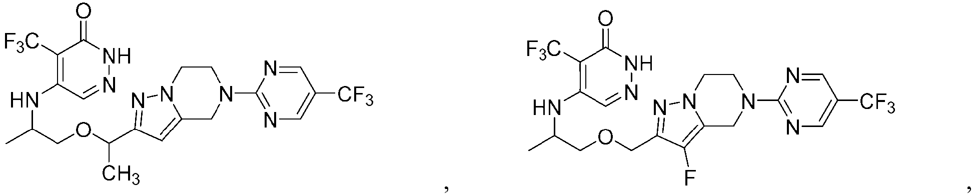 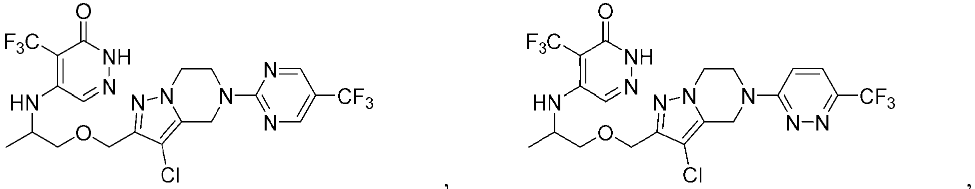 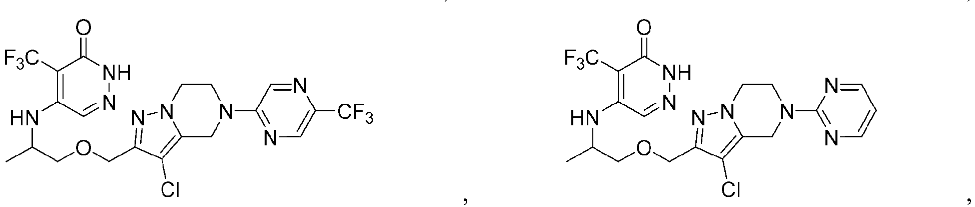 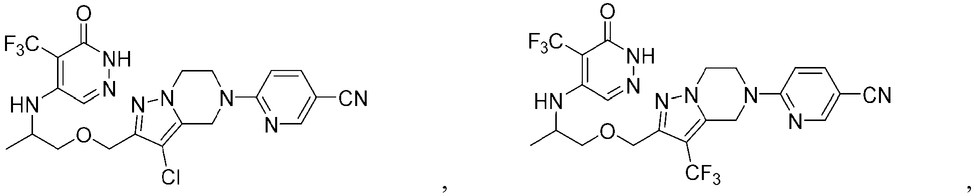 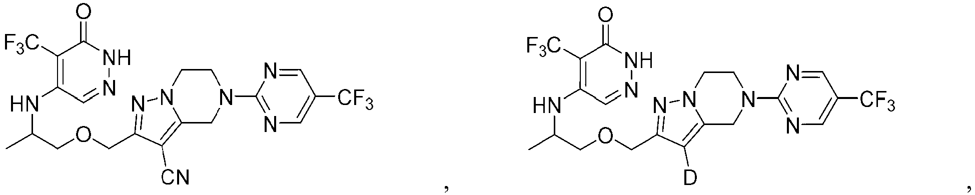 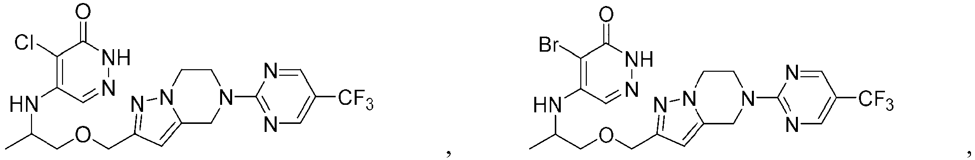 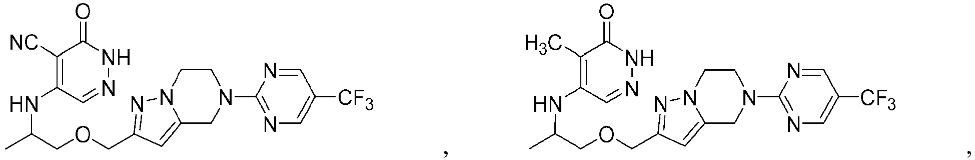 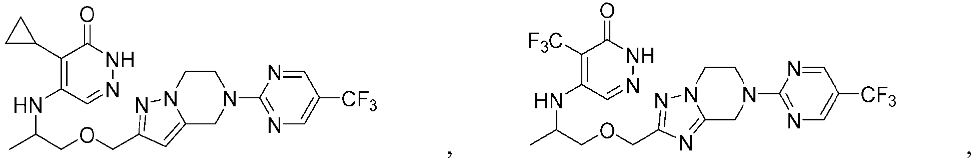 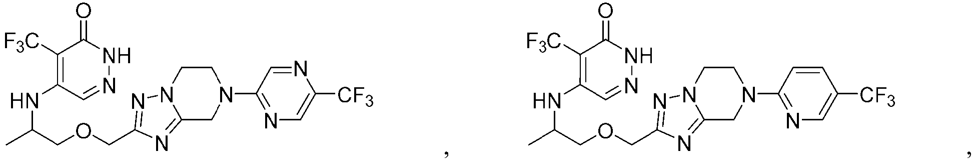 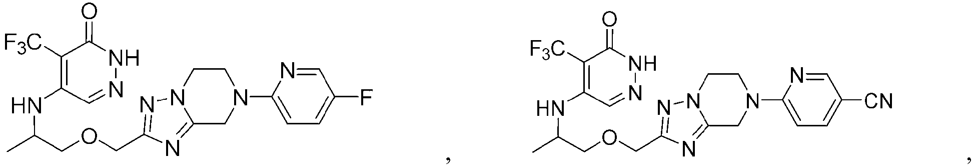 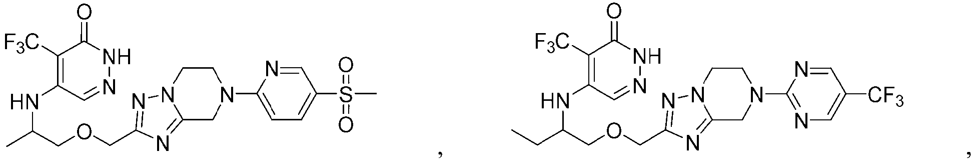 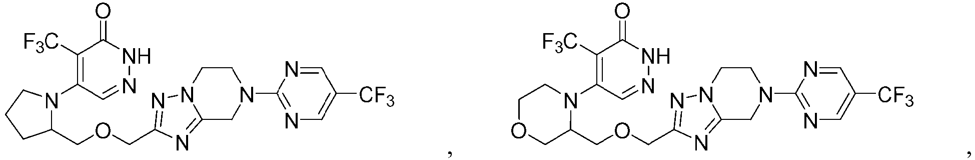 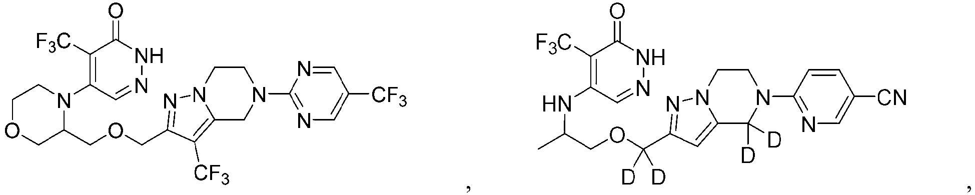 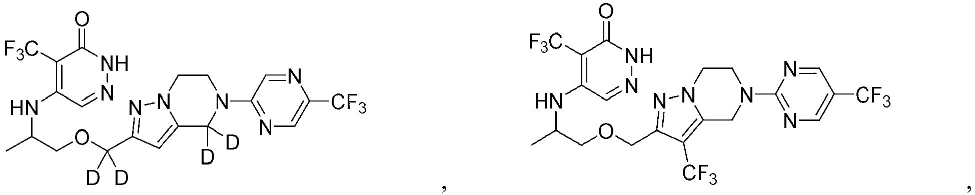 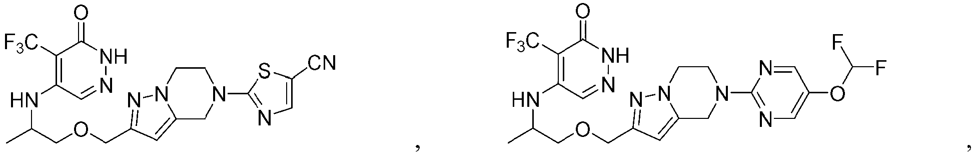 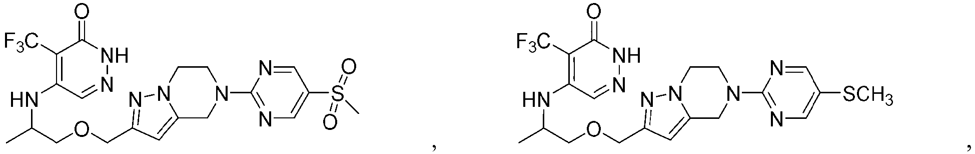 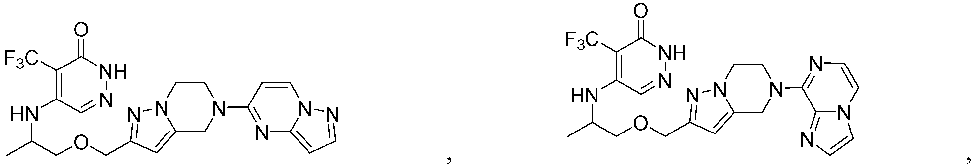 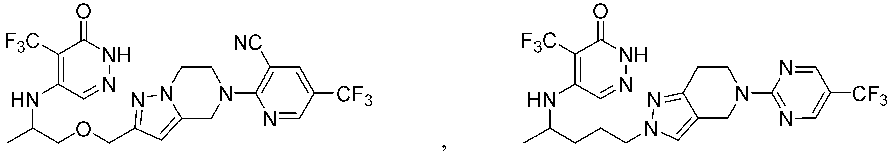 or

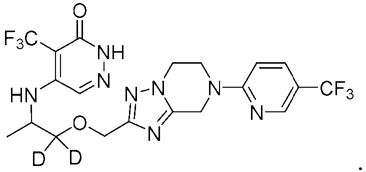

In one or more embodiments, the pharmaceutical preparation of the present invention is characterized in that the pharmaceutical preparation comprises the piperazine derivative, or the stereoisomer, tautomer, deuterated compound or pharmaceutically acceptable salt thereof in a strength of 1-5 mg, 5-10 mg, 10-15 mg, 15-20 mg, 20-25 mg, 25-30 mg, 30-35 mg, 35-40 mg, 40-50 mg, 50-60 mg, 60-70 mg, 70-80 mg, 80-90 mg, 90-100 mg, 100-150 mg, 150-200 mg, 200-250 mg, 250-300 mg, 300-350 mg, 350-400 mg, 400-450 mg, 450-500 mg, 500-600 mg, 600-700 mg, 700-800 mg, 800-900 mg or 900-1000 mg.

In one or more embodiments, the pharmaceutical preparation of the present invention is characterized in that the content of the piperazine derivative, or the stereoisomer, tautomer, deuterated compound or pharmaceutically acceptable salt thereof in the pharmaceutical preparation is 5% - 90% (for example, may be about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or any weight percentage range), preferably 20% - 80%, and more preferably 30% - 70% of the total weight of the pharmaceutical preparation.

In one or more embodiments, the pharmaceutical preparation of the present invention is characterized in that the dosage form of the pharmaceutical preparation is selected from a tablet, a capsule, or a granule.

In one or more embodiments, the pharmaceutical preparation of the present invention is characterized in that the pharmaceutical preparation further comprises one or more of a diluent, a disintegrant, a solubilizer, a glidant, and a lubricant.

In one or more embodiments, the pharmaceutical preparation of the present invention is characterized in that the diluent is selected from one or a combination of more of microcrystalline cellulose, starch, mannitol, lactose, pregelatinized starch, dextrin, sucrose, calcium hydrogen phosphate, and calcium phosphate; and the content of the diluent is 10% - 80% (for example, may be about 10%, about 20%, about 30%, about 40%, about 50%, about 60%, about 70%, about 80%, or any weight percentage range) of the total weight of the pharmaceutical preparation.

In one or more embodiments, the pharmaceutical preparation of the present invention is characterized in that the disintegrant is selected from one or a combination of more of microcrystalline cellulose, croscarmellose sodium, crospovidone, sodium carboxymethyl starch, low-substituted hydroxypropylcellulose, polacrilin potassium, pregelatinized starch, and starch; and the content of the disintegrant is 1% - 60% (for example, may be about 1%, about 5%, about 10%, about 15%, about 20%, about 25%, about 30%, about 35%, about 40%, about 45%, about 50%, about 55%, about 60%, or any weight percentage range), preferably 3% - 20% of the total weight of the pharmaceutical preparation.

In one or more embodiments, the pharmaceutical preparation of the present invention is characterized in that the solubilizer is selected from one or a combination of two of poloxamer, and sodium dodecyl sulphate; and the content of the solubilizer is 2% - 10% (for example, may be about 1%, about 3%, about 4%, about 5%, about 6%, about 7%, about 8%, about 9%, about 10%, or any weight percentage range) of the total weight of the pharmaceutical preparation.

In one or more embodiments, the pharmaceutical preparation of the present invention is characterized in that the glidant is selected from one or a combination of two of colloidal silica and talc; and the content of the glidant is 0.5% - 5% (for example, may be about 0.5%, about 1%, about 1.5%, about 2%, about 2.5%, about 3%, about 3.5%, about 4%, about 4.5%, about 5%, or any weight percentage range) of the total weight of the pharmaceutical preparation.

In one or more embodiments, the pharmaceutical preparation of the present invention is characterized in that the lubricant is selected from one or a combination of more of stearic acid, magnesium stearate, calcium stearate, sodium stearyl fumarate, talc, hydrogenated vegetable oil, magnesium lauryl sulphate, polyethylene glycol, glyceryl behenate, and a sucrose ester; and the content of the lubricant is 0.5% - 5% (for example, may be about 0.5%, about 1%, about 1.5%, about 2%, about 2.5%, about 3%, about 3.5%, about 4%, about 4.5%, about 5%, or any weight percentage range) of the total weight of the pharmaceutical preparation.

One or more embodiments of the present invention provide a preparation method for a pharmaceutical preparation of a piperazine derivative, the preparation method comprising micronizing the piperazine derivative, with the particle size thereof being controlled to 5-100 µm, preferably 5-70 µm, more preferably 5-40 µm; mixing the micronized piperazine derivative with a diluent, a disintegrant and/or a solubilizer to obtain a mixture; subjecting the mixture to dry granulation, and adding a glidant and/or a lubricant to the prepared granules to obtain intermediate granules; then preparing same into a tablet, a capsule, or a granule; and optionally coating the tablet.

One or more embodiments of the present invention provide a preparation method for a pharmaceutical preparation of a piperazine derivative, the preparation method comprising micronizing the piperazine derivative, with the particle size thereof being controlled to 5-100 µm, preferably 5-70 µm, more preferably 5-40 µm; mixing the micronized piperazine derivative with a disintegrant and/or a solubilizer, and then adding a diluent, a glidant and/or a lubricant and mixing same to obtain a mixture; subjecting the mixture to dry granulation, and adding the glidant and/or the lubricant to the prepared granules to obtain intermediate granules; then preparing same into a tablet, a capsule, or a granule; and optionally coating the tablet.

One or more embodiments of the present invention provide a preparation method for a pharmaceutical preparation of a piperazine derivative, the preparation method comprising micronizing the piperazine derivative, with the particle size thereof being controlled to 5-100 µm, preferably 5-70 µm, more preferably 5-40 µm; mixing the micronized piperazine derivative with a diluent and/or a disintegrant, then adding the diluent and mixing same, and then adding a glidant and/or a lubricant and mixing same to obtain a mixture; then preparing same into a tablet, a capsule, or a granule; and optionally coating the tablet.

One or more embodiments of the present invention provide the use of the pharmaceutical preparation of a piperazine derivative of the present invention in the preparation of a drug for the treatment and/or prevention of a cancer.

One or more embodiments of the present invention provide a method for preventing and/or treating a cancer, comprising administering to a patient a therapeutically effective dose of the pharmaceutical preparation of a piperazine derivative of the present invention.

In one or more embodiments, the cancer mentioned in the present invention is selected from a solid tumour, which is selected from breast cancer, central nervous system cancer, uterine cancer, cervical cancer, renal cancer, adrenal cancer, lung cancer, oesophageal cancer, ovarian cancer, pancreatic cancer, liver cancer, prostate cancer, testicular cancer, gastric cancer, head and neck cancer, laryngeal cancer, urinary tract cancer, bladder cancer, colon cancer, rectal cancer, thyroid cancer, bone cancer, epithelial cancer, bile duct cancer, gallbladder cancer, skin cancer, mesothelioma, basal cell carcinoma, adenolike cystic carcinoma, leiomyosarcoma, gastrointestinal stromal tumour, Ewing sarcoma, Kaposi's sarcoma, or advanced solid tumour with PARP7 amplification.

In one or more embodiments, the cancer mentioned in the present invention is selected from a haematological tumour, which is selected from leukaemia, myeloma, and lymphoma. As an example, the haematological tumour includes Hodgkin's lymphoma or non-Hodgkin's lymphoma, multiple myeloma, B-cell lymphoma, small lymphocytic lymphoma, T-cell lymphoma, hairy cell lymphoma, Burkitt lymphoma, acute lymphocytic leukaemia, acute myeloid leukaemia, chronic lymphocytic leukaemia, and chronic myeloid leukaemia.

The lung cancer is preferably non-small cell lung cancer or neuroendocrine lung cancer. The non-small cell lung cancer is preferably lung squamous cell carcinoma or lung adenocarcinoma. The breast cancer is preferably hormone-receptor-positive (HR+) breast cancer. The oesophageal cancer is preferably oesophageal squamous-cell carcinoma or oesophageal adenocarcinoma. The head and neck cancer is preferably head and neck squamous cell carcinoma. The uterine cancer is preferably endometrial cancer. The central nervous system cancer is preferably glioma. The liver cancer is preferably hepatocellular carcinoma. The B-cell lymphoma is preferably diffuse large B-cell lymphoma.

Unless stated to the contrary, the terms used in the specification and claims have the following meanings.

"Stereoisomer" refers to isomers resulting from different spatial arrangements of atoms in a molecule, including cis/trans isomers, enantiomers, and conformers.

The "tautomer" or "tautomeric form" refers to structural isomers of different energies that are interconvertible via a low energy barrier. For example, proton tautomer (also known as prototropic tautomer) includes interconversions via proton migration, such as keto-enol isomerization, imine-enamine isomerization, and lactam-lactim isomerization. The present disclosure includes any tautomer of the compound.

"Pharmaceutically acceptable salt" or "pharmaceutically acceptable salt thereof" refers to a salt of the compound of the present application, which salt maintains the biological effectiveness and characteristics of a free acid or a free base, and is obtained by reacting the free acid with a non-toxic inorganic base or organic base, or reacting the free base with a non-toxic inorganic acid or organic acid.

"Optional", "optionally", "selective", or "selectively" means that the subsequently described event or circumstance may, but not necessarily, occur, and the description includes cases where the event or circumstance occurs and cases where it does not. For example, "heterocyclyl alternatively substituted with alkyl" means that the alkyl may but not necessarily exist, and the description includes the case where the heterocyclyl is substituted with alkyl and the case where the heterocyclyl is not substituted with alkyl.

### DETAILED DESCRIPTION OF THE EMBODIMENTS

The technical solution of the present invention will be explained in detail by the following examples; however, the scope of protection of the present invention includes but is not limited thereto.

The piperazine derivative of the present invention can be prepared by the method in patent WO 2022242750, and in particular, the compound A used in the examples of the present invention is selected from compound 1 in patent WO 2022242750,which has a structure as follows:

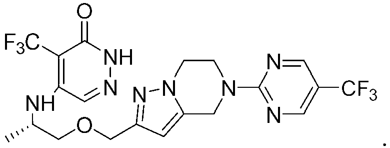

### Example 1

| **Component** | **Unit/mg** | **Percentage/%** |
|---|---|---|
| Compound A | 200 | 45.5 |
| Microcrystalline cellulose | 120 | 27.3 |
| Lactose | 80 | 18.2 |
| Croscarmellose sodium | 30 | 6.8 |
| Colloidal silica | 5 | 1.1 |
| Magnesium stearate | 5 | 1.1 |

### Preparation method:

1) Compound A was micronized using a jet pulverization process, with a particle size X90 = 37.69 µm; and prescribed amounts of compound A, lactose, and croscarmellose sodium were weighed, sieved through a 40-mesh sieve twice, and mixed for 5 min to obtain mixture 1.
2) A prescribed amount of microcrystalline cellulose was added to mixture 1 and mixed for 5 min to obtain mixture 2.
3) Prescribed amounts of colloidal silica and magnesium stearate were added to mixture 2 and mixed for 5 min to obtain an intermediate powder.
4) Tablet compression was carried out using a φ 10 mm die to produce tablets with a strength of 200 mg, with the tablet weight being controlled at 440 mg ± 5%.

### Example 2

| **Component** | **Unit/mg** | **Percentage/%** |
|---|---|---|
| Compound A | 200 | 45.5 |
| Microcrystalline cellulose | 120 | 27.3 |
| Lactose | 80 | 18.2 |
| Croscarmellose sodium | 30 | 6.8 |
| Colloidal silica | 5 | 1.1 |
| Magnesium stearate | 5 | 1.1 |

### Preparation method:

The same as in **Example 1,** with a particle size X90 = 26.16 µm.

### Example 3

| **Component** | **Unit/mg** | **Percentage/%** |
|---|---|---|
| Compound A | 200 | 45.5 |
| Microcrystalline cellulose | 120 | 27.3 |
| Lactose | 80 | 18.2 |
| Croscarmellose sodium | 30 | 6.8 |
| Colloidal silica | 5 | 1.1 |
| Magnesium stearate | 5 | 1.1 |

### Preparation method:

The same as in **Example 1,** with a particle size X90 = 12.07 µm.

### Example 4

| **Component** | **Unit/mg** | **Percentage/%** |
|---|---|---|
| Compound A | 200 | 45.5 |
| Microcrystalline cellulose | 120 | 27.3 |
| Lactose | 80 | 18.2 |
| Croscarmellose sodium | 30 | 6.8 |
| Colloidal silica | 5 | 1.1 |
| Magnesium stearate | 5 | 1.1 |

### Preparation method:

The same as in **Example 1,** with a particle size X90 = 72.98 µm.

### Example 5

| **Component** | **Unit/mg** | **Percentage/%** |
|---|---|---|
| Compound A | 200 | 45.5 |
| Microcrystalline cellulose | 200 | 45.5 |
| Croscarmellose sodium | 30 | 6.8 |
| Colloidal silica | 5 | 1.1 |
| Magnesium stearate | 5 | 1.1 |

### Preparation method:

1) Compound A was micronized using a jet pulverization process, with a particle size X90 being controlled to ≤ 40 µm, and sieved through a 60-mesh sieve; and microcrystalline cellulose, croscarmellose sodium, colloidal silica, and magnesium stearate were sieved through a 40-mesh sieve, respectively.
2) Prescribed amounts of compound A, microcrystalline cellulose, and croscarmellose sodium were weighed, sieved through a 40-mesh sieve twice, and mixed for 5 min to obtain mixture 1.
3) Mixture 1 was subjected to dry granulation, with a roller compaction speed controlled at 3-5 Hz, a granulation speed controlled at 15-20 Hz, and an oil pump pressure controlled at 30-80 kg/cm², to obtain granules 1.
4) Prescribed amounts of colloidal silica and magnesium stearate were added to granules 1 and mixed for 5 min to obtain intermediate granules 2. The intermediate granules 2 were analysed by HPLC, and the content of compound A was found to be 43.0% - 48.0%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
5) Based on the content of intermediate granules 2, tablet compression was carried out using a φ 10 mm die to produce tablets with a strength of 200 mg, where the tablets were controlled to have a weight variation of ± 5% and a hardness of 70-110 N.

### Example 6

| **Component** | **Unit/mg** | **Percentage/%** |
|---|---|---|
| Compound A | 200 | 45.5 |
| Microcrystalline cellulose | 120 | 27.3 |
| Lactose | 80 | 18.2 |
| Croscarmellose sodium | 30 | 6.8 |
| Colloidal silica | 5 | 1.1 |
| Magnesium stearate | 5 | 1.1 |

### Preparation method:

1) Compound A was micronized using a jet pulverization process, with a particle size X90 being controlled to ≤ 40 µm, and sieved through a 60-mesh sieve; and microcrystalline cellulose, lactose, croscarmellose sodium, colloidal silica, and magnesium stearate were sieved through a 40-mesh sieve, respectively.
2) Prescribed amounts of compound A, microcrystalline cellulose, lactose, and croscarmellose sodium were weighed, sieved through a 40-mesh sieve twice, and mixed for 5 min to obtain mixture 1.
3) Mixture 1 was subjected to dry granulation, with a roller compaction speed controlled at 3-5 Hz, a granulation speed controlled at 15-20 Hz, and an oil pump pressure controlled at 30-80 kg/cm², to obtain granules 1.
4) Prescribed amounts of colloidal silica and magnesium stearate were added to granules 1 and mixed for 5 min to obtain intermediate granules 2. The intermediate granules 2 were analysed by HPLC, and the content of compound A was found to be 43.0% - 48.0%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
5) Based on the content of intermediate granules 2, tablet compression was carried out using a φ 10 mm die to produce tablets with a strength of 200 mg, where the tablets were controlled to have a weight variation of ± 5% and a hardness of 70-110 N.

### Example 7

| **Component** | **Unit/mg** | **Percentage/%** |
|---|---|---|
| Compound A | 200 | 56.8 |
| Microcrystalline cellulose | 120 | 34.1 |
| Croscarmellose sodium | 24 | 6.8 |
| Colloidal silica | 4 | 1.1 |
| Magnesium stearate | 4 | 1.1 |

### Preparation method:

The same as in **Example 5.** The intermediate granules 2 were analysed by HPLC, and the content of compound A was found to be 54.3% - 59.3%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.

### Example 8

| **Component** | **Unit/mg** | **Percentage/%** |
|---|---|---|
| Compound A | 200 | 50.5 |
| Microcrystalline cellulose | 160 | 40.4 |
| Croscarmellose sodium | 27 | 6.8 |
| Colloidal silica | 4.5 | 1.1 |
| Magnesium stearate | 4.5 | 1.1 |

### Preparation method:

The same as in **Example 5.** The intermediate granules 2 were analysed by HPLC, and the content of compound A was found to be 48.0% - 53.0%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.

### Example 9

| **Component** | **Unit/mg** | **Percentage/%** |
|---|---|---|
| Compound A | 200 | 45.5 |
| Microcrystalline cellulose | 200 | 45.5 |
| Crospovidone | 30 | 6.8 |
| Colloidal silica | 5 | 1.1 |
| Magnesium stearate | 5 | 1.1 |

### Preparation method:

1) Compound A was micronized using a jet pulverization process, with a particle size X90 being controlled to ≤ 40 µm, and sieved through a 60-mesh sieve; and microcrystalline cellulose, crospovidone, colloidal silica, and magnesium stearate were sieved through a 40-mesh sieve, respectively.
2) Prescribed amounts of compound A, microcrystalline cellulose, and crospovidone were weighed, sieved through a 40-mesh sieve twice, and mixed for 5 min to obtain mixture 1.
3) Mixture 1 was subjected to dry granulation, with a roller compaction speed controlled at 3-5 Hz, a granulation speed controlled at 15-20 Hz, and an oil pump pressure controlled at 30-80 kg/cm², to obtain granules 1.
4) Prescribed amounts of colloidal silica and magnesium stearate were added to granules 1 and mixed for 5 min to obtain intermediate granules 2. The intermediate granules 2 were analysed by HPLC, and the content of compound A was found to be 43.0% - 48.0%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
5) Based on the content of intermediate granules 2, tablet compression was carried out using a φ 10 mm die to produce tablets with a strength of 200 mg, where the tablets were controlled to have a weight variation of ± 5% and a hardness of 70-110 N.

### Example 10

| **Component** | **Unit/mg** | **Percentage/%** |
|---|---|---|
| Compound A | 200 | 45.5 |
| Microcrystalline cellulose | 200 | 45.5 |
| Sodium carboxymethyl starch | 30 | 6.8 |
| Colloidal silica | 5 | 1.1 |
| Magnesium stearate | 5 | 1.1 |

### Preparation method:

1) Compound A was micronized using a jet pulverization process, with a particle size X90 being controlled to ≤ 40 µm, and sieved through a 60-mesh sieve; and microcrystalline cellulose, sodium carboxymethyl starch, colloidal silica, and magnesium stearate were sieved through a 40-mesh sieve, respectively.
2) Prescribed amounts of compound A, microcrystalline cellulose, and sodium carboxymethyl starch were weighed, sieved through a 40-mesh sieve twice, and mixed for 5 min to obtain mixture 1.
3) Mixture 1 was subjected to dry granulation, with a roller compaction speed controlled at 3-5 Hz, a granulation speed controlled at 15-20 Hz, and an oil pump pressure controlled at 30-80 kg/cm², to obtain granules 1.
4) Prescribed amounts of colloidal silica and magnesium stearate were added to granules 1 and mixed for 5 min to obtain intermediate granules 2. The intermediate granules 2 were analysed by HPLC, and the content of compound A was found to be 43.0% - 48.0%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
5) Based on the content of intermediate granules 2, tablet compression was carried out using a φ 10 mm die to produce tablets with a strength of 200 mg, where the tablets were controlled to have a weight variation of ± 5% and a hardness of 70-110 N.

### Example 11

| **Component** | **Unit/mg** | **Percentage/%** |
|---|---|---|
| Compound A | 200 | 45.5 |
| Microcrystalline cellulose | 188 | 42.7 |
| Crospovidone | 20 | 4.6 |
| Poloxamer | 22 | 5.0 |
| Colloidal silica | 5 | 1.1 |
| Magnesium stearate | 5 | 1.1 |

### Preparation method:

1) Compound A was micronized using a jet pulverization process, with a particle size X90 being controlled to ≤ 40 µm, and sieved through a 60-mesh sieve; poloxamer was sieved through a 60-mesh sieve; and microcrystalline cellulose, crospovidone, colloidal silica, and magnesium stearate were sieved through a 40-mesh sieve, respectively.
2) Prescribed amounts of compound A and poloxamer were weighed and sieved through a 40-mesh sieve twice, and a prescribed amount of crospovidone was added and sieved through a 40-mesh sieve twice to obtain mixture 1.
3) A prescribed amount of microcrystalline cellulose was added to mixture 1 and mixed for 10 min, and 1/2 of a prescribed amount of magnesium stearate and 1/2 of a prescribed amount of colloidal silica were added and mixed for 5 min to obtain mixture 2.
4) Mixture 2 was subjected to dry granulation, with a roller compaction speed being 4-6 Hz, a granulation speed being 15-20 Hz, and an oil pump pressure being 30-80 kg/cm², to obtain granules 1.
5) The remaining prescribed amounts of magnesium stearate and colloidal silica were added to granules 1 and mixed for 5 min to obtain intermediate granules 2. The intermediate granules 2 were analysed by HPLC, and the content of compound A was found to be 43.0% - 48.0%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
6) Based on the content of intermediate granules 2, tablet compression was carried out using a φ 10 mm die to produce tablets with a strength of 200 mg, where the tablets were controlled to have a weight variation of ± 5% and a hardness of 70-110 N.

### Example 12

| **Component** | **Unit/mg** | **Percentage/%** |
|---|---|---|
| Compound A | 200 | 50.0 |
| Microcrystalline cellulose | 140 | 35.0 |
| Crospovidone | 20 | 5.0 |
| Poloxamer | 32 | 8.0 |
| Colloidal silica | 4 | 1.0 |
| Magnesium stearate | 4 | 1.0 |

### Preparation method:

The same as in **Example 11.** The intermediate granules 2 were analysed by HPLC, and the content of compound A was found to be 47.5% - 52.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.

### Example 13

| **Component** | **Unit/mg** | **Percentage/%** |
|---|---|---|
| Compound A | 200 | 50.0 |
| Microcrystalline cellulose | 145 | 36.3 |
| Crospovidone | 27 | 6.8 |
| Poloxamer | 20 | 5.0 |
| Colloidal silica | 4 | 1.0 |
| Magnesium stearate | 4 | 1.0 |

### Preparation method:

The same as in **Example 11.** The intermediate granules 2 were analysed by HPLC, and the content of compound A was found to be 47.5% - 52.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.

### Example 14

| **Component** | **Unit/mg** | **Percentage/%** |
|---|---|---|
| Compound A | 200 | 50.0 |
| Microcrystalline cellulose | 145 | 36.3 |
| Crospovidone | 27 | 6.8 |
| Poloxamer | 20 | 5.0 |
| Colloidal silica | 4 | 1.0 |
| Magnesium stearate | 4 | 1.0 |
| Film coating premix | 10 | 2.5 |

### Preparation method:

1) Compound A was micronized using a jet pulverization process, with a particle size X90 being controlled to ≤ 40 µm, and sieved through a 60-mesh sieve; and poloxamer was sieved through a 60-mesh sieve, and microcrystalline cellulose, crospovidone, colloidal silica, and magnesium stearate were sieved through a 40-mesh sieve, respectively.
2) Prescribed amounts of compound A and poloxamer were weighed and sieved through a 40-mesh sieve twice, and a prescribed amount of crospovidone was added and sieved through a 40-mesh sieve twice to obtain mixture 1.
3) A prescribed amount of microcrystalline cellulose was added to mixture 1 and mixed for 10 min, and 1/2 of a prescribed amount of magnesium stearate and 1/2 of a prescribed amount of colloidal silica were added and mixed for 5 min to obtain mixture 2.
4) Mixture 2 was subjected to dry granulation, with a roller compaction speed being 4-6 Hz, a granulation speed being 15-20 Hz, and an oil pump pressure being 30-80 kg/cm², to obtain granules 1.
5) The remaining prescribed amounts of magnesium stearate and colloidal silica were added to granules 1 and mixed for 5 min to obtain intermediate granules 2. The intermediate granules 2 were analysed by HPLC, and the content of compound A was found to be 47.5% - 52.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
6) Based on the content of intermediate granules 2, tablet compression was carried out using a φ 10 mm die to produce tablets with a strength of 200 mg, where the tablets were controlled to have a weight variation of ± 5% and a hardness of 70-110 N.
7) The prepared tablets were coated with a coating weight gain of about 2.0-5.0%.

### Example 15

| **Component** | **Unit/mg** | **Percentage/%** |
|---|---|---|
| Compound A | 50 | 50.0 |
| Microcrystalline cellulose | 36.25 | 36.3 |
| Crospovidone | 6.75 | 6.8 |
| Poloxamer | 5 | 5.0 |
| Colloidal silica | 1 | 1.0 |
| Magnesium stearate | 1 | 1.0 |
| Film coating premix | 2.5 | 2.5 |

### Preparation method:

1) Compound A was micronized using a jet pulverization process, with a particle size X90 being controlled to ≤ 40 µm, and sieved through a 60-mesh sieve; and poloxamer was sieved through a 60-mesh sieve, and microcrystalline cellulose, crospovidone, colloidal silica, and magnesium stearate were sieved through a 40-mesh sieve, respectively.
2) Prescribed amounts of compound A and poloxamer were weighed and sieved through a 40-mesh sieve twice, and a prescribed amount of crospovidone was added and sieved through a 40-mesh sieve twice to obtain mixture 1.
3) A prescribed amount of microcrystalline cellulose was added to mixture 1 and mixed for 10 min, and 1/2 of a prescribed amount of magnesium stearate and 1/2 of a prescribed amount of colloidal silica were added and mixed for 5 min to obtain mixture 2.
4) Mixture 2 was subjected to dry granulation, with a roller compaction speed being 4-6 Hz, a granulation speed being 15-20 Hz, and an oil pump pressure being 30-80 kg/cm², to obtain granules 1.
5) The remaining prescribed amounts of magnesium stearate and colloidal silica were added to granules 1 and mixed for 5 min to obtain intermediate granules 2. The intermediate granules 2 were analysed by HPLC, and the content of compound A was found to be 47.5% - 52.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
6) Based on the content of intermediate granules 2, tablet compression was carried out using a φ 6 mm die to produce tablets with a strength of 50 mg, where the tablets were controlled to have a weight variation of ± 5% and a hardness of 50-80 N.
7) The prepared tablets were coated with a coating weight gain of about 2.0-5.0%.

### Example 16

| **Component** | **Unit/mg** | **Percentage/%** |
|---|---|---|
| Compound A | 100 | 50.0 |
| Microcrystalline cellulose | 72.5 | 36.3 |
| Crospovidone | 13.5 | 6.8 |
| Poloxamer | 10 | 5.0 |
| Colloidal silica | 2 | 1.0 |
| Magnesium stearate | 2 | 1.0 |
| Film coating premix | 5 | 2.5 |

### Preparation method:

1) Compound A was micronized using a jet pulverization process, with a particle size X90 being controlled to ≤ 40 µm, and sieved through a 60-mesh sieve; and poloxamer was sieved through a 60-mesh sieve, and microcrystalline cellulose, crospovidone, colloidal silica, and magnesium stearate were sieved through a 40-mesh sieve, respectively.
2) Prescribed amounts of compound A and poloxamer were weighed and sieved through a 40-mesh sieve twice, and a prescribed amount of crospovidone was added and sieved through a 40-mesh sieve twice to obtain mixture 1.
3) A prescribed amount of microcrystalline cellulose was added to mixture 1 and mixed for 10 min, and 1/2 of a prescribed amount of magnesium stearate and 1/2 of a prescribed amount of colloidal silica were added and mixed for 5 min to obtain mixture 2.
4) Mixture 2 was subjected to dry granulation, with a roller compaction speed being 4-6 Hz, a granulation speed being 15-20 Hz, and an oil pump pressure being 30-80 kg/cm², to obtain granules 1.
5) The remaining prescribed amounts of magnesium stearate and colloidal silica were added to granules 1 and mixed for 5 min to obtain intermediate granules 2. The intermediate granules 2 were analysed by HPLC, and the content of compound A was found to be 47.5% - 52.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
6) Based on the content of intermediate granules 2, tablet compression was carried out using a φ 8 mm die to produce tablets with a strength of 100 mg, where the tablets were controlled to have a weight variation of ± 5% and a hardness of 60-100 N.
7) The prepared tablets were coated with a coating weight gain of about 2.0-5.0%.

### Example 17

| **Component** | **Unit/mg** | **Percentage/%** |
|---|---|---|
| Compound A | 120 | 30.0 |
| Microcrystalline cellulose | 225 | 56.2 |
| Crospovidone | 27 | 6.8 |
| Poloxamer | 20 | 5.0 |
| Colloidal silica | 4 | 1.0 |
| Magnesium stearate | 4 | 1.0 |
| Film coating premix | 10 | 2.5 |

### Preparation method:

1) Compound A was micronized using a jet pulverization process, with a particle size X90 being controlled to ≤ 40 µm, and sieved through a 60-mesh sieve; and poloxamer was sieved through a 60-mesh sieve, and microcrystalline cellulose, crospovidone, colloidal silica, and magnesium stearate were sieved through a 40-mesh sieve, respectively.
2) Prescribed amounts of compound A and poloxamer were weighed and sieved through a 40-mesh sieve twice, and a prescribed amount of crospovidone was added and sieved through a 40-mesh sieve twice to obtain mixture 1.
3) A prescribed amount of microcrystalline cellulose was added to mixture 1 and mixed for 10 min to obtain mixture 2.
4) Mixture 2 was subjected to dry granulation, with a roller compaction speed being 4-6 Hz, a granulation speed being 15-20 Hz, and an oil pump pressure being 30-80 kg/cm², to obtain granules 1.
5) Prescribed amounts of magnesium stearate and colloidal silica were added to granules 1 and mixed for 5 min to obtain intermediate granules 2. The intermediate granules 2 were analysed by HPLC, and the content of compound A was found to be 27.5% - 32.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
6) Based on the content of intermediate granules 2, tablet compression was carried out using a φ 10 mm die to produce tablets with a strength of 120 mg, where the tablets were controlled to have a weight variation of ± 5% and a hardness of 70-110 N.
7) The prepared tablets were coated with a coating weight gain of about 2.0-5.0%.

### Example 18

| **Component** | **Unit/mg** | **Percentage/%** |
|---|---|---|
| Compound A | 280 | 70.0 |
| Microcrystalline cellulose | 65 | 16.2 |
| Crospovidone | 27 | 6.8 |
| Poloxamer | 20 | 5.0 |
| Colloidal silica | 4 | 1.0 |
| Magnesium stearate | 4 | 1.0 |
| Film coating premix | 10 | 2.5 |

### Preparation method:

1) Compound A was micronized using a jet pulverization process, with a particle size X90 being controlled to ≤ 40 µm, and sieved through a 60-mesh sieve; and poloxamer was sieved through a 60-mesh sieve, and microcrystalline cellulose, crospovidone, colloidal silica, and magnesium stearate were sieved through a 40-mesh sieve, respectively.
2) Prescribed amounts of compound A and poloxamer were weighed and sieved through a 40-mesh sieve twice, and a prescribed amount of crospovidone was added and sieved through a 40-mesh sieve twice to obtain mixture 1.
3) A prescribed amount of microcrystalline cellulose was added to mixture 1 and mixed for 10 min, and 1/2 of a prescribed amount of magnesium stearate and 1/2 of a prescribed amount of colloidal silica were added and mixed for 5 min to obtain mixture 2.
4) Mixture 2 was subjected to dry granulation, with a roller compaction speed being 4-6 Hz, a granulation speed being 15-20 Hz, and an oil pump pressure being 30-80 kg/cm², to obtain granules 1.
5) The remaining prescribed amounts of magnesium stearate and colloidal silica were added to granules 1 and mixed for 5 min to obtain intermediate granules 2. The intermediate granules 2 were analysed by HPLC, and the content of compound A was found to be 67.5% - 72.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
6) Based on the content of intermediate granules 2, tablet compression was carried out using a φ 10 mm die to produce tablets with a strength of 280 mg, where the tablets were controlled to have a weight variation of ± 5% and a hardness of 70-110 N.
7) The prepared tablets were coated with a coating weight gain of about 2.0-5.0%.

### Example 19

| **Component** | **Unit/mg** | **Percentage/%** |
|---|---|---|
| Compound A | 200 | 50.0 |
| Microcrystalline cellulose | 140 | 35.0 |
| Crospovidone | 12 | 3.0 |
| Poloxamer | 40 | 10.0 |
| Colloidal silica | 4 | 1.0 |
| Magnesium stearate | 4 | 1.0 |
| Film coating premix | 10 | 2.5 |

### Preparation method:

The same as in **Example 14.**

### Example 20

| **Component** | **Unit/mg** | **Percentage/%** |
|---|---|---|
| Compound A | 200 | 50.0 |
| Microcrystalline cellulose | 104 | 26.0 |
| Crospovidone | 80 | 20.0 |
| Sodium dodecyl sulphate | 8 | 2.0 |
| Colloidal silica | 4 | 1.0 |
| Magnesium stearate | 4 | 1.0 |
| Film coating premix | 10 | 2.5 |

### Preparation method:

The same as in **Example 14.**

### Example 21

| **Component** | **Unit/mg** | **Percentage/%** |
|---|---|---|
| Compound A | 200 | 50.0 |
| Microcrystalline cellulose | 139 | 34.7 |
| Crospovidone | 27 | 6.8 |
| Poloxamer | 20 | 5.0 |
| Colloidal silica | 2 | 0.5 |
| Magnesium stearate | 12 | 3.0 |
| Film coating premix | 10 | 2.5 |

### Preparation method:

The same as in **Example 14.**

### Example 22

| **Component** | **Unit/mg** | **Percentage/%** |
|---|---|---|
| Compound A | 200 | 50.0 |
| Microcrystalline cellulose | 139 | 34.7 |
| Crospovidone | 27 | 6.8 |
| Poloxamer | 20 | 5.0 |
| Colloidal silica | 12 | 3.0 |
| Magnesium stearate | 2 | 0.5 |
| Film coating premix | 10 | 2.5 |

### Preparation method:

The same as in **Example 14.**

### Example 23

| **Component** | **Unit/mg** | **Percentage/%** |
|---|---|---|
| Compound A | 200 | 50.0 |
| Starch | 145 | 36.3 |
| Low-substituted hydroxypropylcellulose | 27 | 6.8 |
| Poloxamer | 20 | 5.0 |
| Colloidal silica | 4 | 1.0 |
| Magnesium stearate | 4 | 1.0 |
| Film coating premix | 10 | 2.5 |

### Preparation method:

1) Compound A was micronized using a jet pulverization process, with a particle size X90 being controlled to ≤ 40 µm, and sieved through a 60-mesh sieve; and poloxamer was sieved through a 60-mesh sieve, and starch, microcrystalline cellulose, colloidal silica, and magnesium stearate were sieved through a 40-mesh sieve, respectively.
2) Prescribed amounts of compound A and poloxamer were weighed and sieved through a 40-mesh sieve twice, and a prescribed amount of microcrystalline cellulose was added and sieved through a 40-mesh sieve twice to obtain mixture 1.
3) A prescribed amount of starch was added to mixture 1 and mixed for 10 min, and 1/2 of a prescribed amount of magnesium stearate and 1/2 of a prescribed amount of colloidal silica were added and mixed for 5 min to obtain mixture 2.
4) Mixture 2 was subjected to dry granulation, with a roller compaction speed being 4-6 Hz, a granulation speed being 15-20 Hz, and an oil pump pressure being 30-80 kg/cm², to obtain granules 1.
5) The remaining prescribed amounts of magnesium stearate and colloidal silica were added to granules 1 and mixed for 5 min to obtain intermediate granules 2. The intermediate granules 2 were analysed by HPLC, and the content of compound A was found to be 47.5% - 52.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
6) Based on the content of intermediate granules 2, tablet compression was carried out using a φ 10 mm die to produce tablets with a strength of 200 mg, where the tablets were controlled to have a weight variation of ± 5% and a hardness of 70-110 N.
7) The prepared tablets were coated with a coating weight gain of about 2.0-5.0%.

### Example 24

| **Component** | **Unit/mg** | **Percentage/%** |
|---|---|---|
| Compound A | 200 | 50.0 |
| Mannitol | 145 | 36.3 |
| Microcrystalline cellulose | 27 | 6.8 |
| Poloxamer | 20 | 5.0 |
| Colloidal silica | 4 | 1.0 |
| Magnesium stearate | 4 | 1.0 |
| Film coating premix | 10 | 2.5 |

### Preparation method:

1) Compound A was micronized using a jet pulverization process, with a particle size X90 being controlled to ≤ 40 µm, and sieved through a 60-mesh sieve; and poloxamer was sieved through a 60-mesh sieve, and mannitol, low-substituted hydroxypropylcellulose, colloidal silica, and magnesium stearate were sieved through a 40-mesh sieve, respectively.
2) Prescribed amounts of compound A and poloxamer were weighed and sieved through a 40-mesh sieve twice, and a prescribed amount of low-substituted hydroxypropylcellulose was added and sieved through a 40-mesh sieve twice to obtain mixture 1.
3) A prescribed amount of mannitol was added to mixture 1 and mixed for 10 min, and 1/2 of a prescribed amount of magnesium stearate and 1/2 of a prescribed amount of colloidal silica were added and mixed for 5 min to obtain mixture 2.
4) Mixture 2 was subjected to dry granulation, with a roller compaction speed being 4-6 Hz, a granulation speed being 15-20 Hz, and an oil pump pressure being 30-80 kg/cm², to obtain granules 1.
5) The remaining prescribed amounts of magnesium stearate and colloidal silica were added to granules 1 and mixed for 5 min to obtain intermediate granules 2. The intermediate granules 2 were analysed by HPLC, and the content of compound A was found to be 47.5% - 52.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
6) Based on the content of intermediate granules 2, tablet compression was carried out using a φ 10 mm die to produce tablets with a strength of 200 mg, where the tablets were controlled to have a weight variation of ± 5% and a hardness of 70-110 N.
7) The prepared tablets were coated with a coating weight gain of about 2.0-5.0%.

### Example 25

| **Component** | **Unit/mg** | **Percentage/%** |
|---|---|---|
| Compound A | 200 | 50.0 |
| Microcrystalline cellulose | 145 | 36.3 |
| Crospovidone | 27 | 6.8 |
| Sodium dodecyl sulphate | 20 | 5.0 |
| Talc | 4 | 1.0 |
| Sodium stearyl fumarate | 4 | 1.0 |
| Film coating premix | 10 | 2.5 |

### Preparation method:

1) Compound A was micronized using a jet pulverization process, with a particle size X90 being controlled to ≤ 40 µm, and sieved through a 60-mesh sieve; and sodium dodecyl sulphate was sieved through a 60-mesh sieve, and microcrystalline cellulose, crospovidone, talc, and sodium stearyl fumarate were sieved through a 40-mesh sieve, respectively.
2) Prescribed amounts of compound A and sodium dodecyl sulphate were weighed and sieved through a 40-mesh sieve twice, and a prescribed amount of crospovidone was added and sieved through a 40-mesh sieve twice to obtain mixture 1.
3) A prescribed amount of microcrystalline cellulose was added to mixture 1 and mixed for 10 min, and 1/2 of a prescribed amount of sodium stearyl fumarate and 1/2 of a prescribed amount of talc were added and mixed for 5 min to obtain mixture 2.
4) Mixture 2 was subjected to dry granulation, with a roller compaction speed being 4-6 Hz, a granulation speed being 15-20 Hz, and an oil pump pressure being 30-80 kg/cm², to obtain granules 1.
5) The remaining prescribed amounts of sodium stearyl fumarate and talc were added to granules 1 and mixed for 5 min to obtain intermediate granules 2. The intermediate granules 2 were analysed by HPLC, and the content of compound A was found to be 47.5% - 52.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
6) Based on the content of intermediate granules 2, tablet compression was carried out using a φ 10 mm die to produce tablets with a strength of 200 mg, where the tablets were controlled to have a weight variation of ± 5% and a hardness of 70-110 N.
7) The prepared tablets were coated with a coating weight gain of about 2.0-5.0%.

### Example 26

| **Component** | **Unit/mg** | **Percentage/%** |
|---|---|---|
| Compound A | 200 | 50.0 |
| Pregelatinized starch | 145 | 36.3 |
| Croscarmellose sodium | 27 | 6.8 |
| Poloxamer | 20 | 5.0 |
| Colloidal silica | 4 | 1.0 |
| Stearic acid | 4 | 1.0 |
| Film coating premix | 10 | 2.5 |

### Preparation method:

1) Compound A was micronized using a jet pulverization process, with a particle size X90 being controlled to ≤ 40 µm, and sieved through a 60-mesh sieve; and poloxamer was sieved through a 60-mesh sieve, and pregelatinized starch, starch, colloidal silica, and stearic acid were sieved through a 40-mesh sieve, respectively.
2) Prescribed amounts of compound A and poloxamer were weighed and sieved through a 40-mesh sieve twice, and a prescribed amount of starch was added and sieved through a 40-mesh sieve twice to obtain mixture 1.
3) A prescribed amount of pregelatinized starch was added to mixture 1 and mixed for 10 min, and 1/2 of a prescribed amount of stearic acid and 1/2 of a prescribed amount of colloidal silica were added and mixed for 5 min to obtain mixture 2.
4) Mixture 2 was subjected to dry granulation, with a roller compaction speed being 4-6 Hz, a granulation speed being 15-20 Hz, and an oil pump pressure being 30-80 kg/cm², to obtain granules 1.
5) The remaining prescribed amounts of stearic acid and colloidal silica were added to granules 1 and mixed for 5 min to obtain intermediate granules 2. The intermediate granules 2 were analysed by HPLC, and the content of compound A was found to be 47.5% - 52.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
6) Based on the content of intermediate granules 2, tablet compression was carried out using a φ 10 mm die to produce tablets with a strength of 200 mg, where the tablets were controlled to have a weight variation of ± 5% and a hardness of 70-110 N.
7) The prepared tablets were coated with a coating weight gain of about 2.0-5.0%.

### Example 27

| **Component** | **Unit/mg** | **Percentage/%** |
|---|---|---|
| Compound A | 200 | 50.0 |
| Microcrystalline cellulose | 165 | 41.2 |
| Crospovidone | 27 | 6.8 |
| Colloidal silica | 4 | 1.0 |
| Magnesium stearate | 4 | 1.0 |
| Film coating premix | 10 | 2.5 |

### Preparation method:

1) Compound A was micronized using a jet pulverization process, with a particle size X90 being controlled to ≤ 40 µm, and sieved through a 60-mesh sieve; and microcrystalline cellulose, crospovidone, colloidal silica, and magnesium stearate were sieved through a 40-mesh sieve, respectively.
2) Prescribed amounts of compound A, microcrystalline cellulose, and crospovidone were weighed, sieved through a 40-mesh sieve twice, and mixed for 5 min to obtain mixture 1.
3) Mixture 1 was subjected to dry granulation, with a roller compaction speed controlled at 3-5 Hz, a granulation speed controlled at 15-20 Hz, and an oil pump pressure controlled at 30-80 kg/cm², to obtain granules 1.
4) Prescribed amounts of colloidal silica and magnesium stearate were added to granules 1 and mixed for 5 min to obtain intermediate granules 2. The intermediate granules 2 were analysed by HPLC, and the content of compound A was found to be 47.5% - 52.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
5) Based on the content of intermediate granules 2, tablet compression was carried out using a φ 10 mm die to produce tablets with a strength of 200 mg, where the tablets were controlled to have a weight variation of ± 5% and a hardness of 70-110 N.
6) The prepared tablets were coated with a coating weight gain of about 2.0-5.0%.

### Example 28

| **Component** | **Unit/mg** | **Percentage/%** |
|---|---|---|
| Compound A | 200 | 50.0 |
| Microcrystalline cellulose | 100 | 25.0 |
| Lactose | 65 | 16.3 |
| Crospovidone | 27 | 6.8 |
| Colloidal silica | 4 | 1.0 |
| Magnesium stearate | 4 | 1.0 |
| Film coating premix | 10 | 2.5 |

### Preparation method:

1) Compound A was micronized using a jet pulverization process, with a particle size X90 being controlled to ≤ 40 µm, and sieved through a 60-mesh sieve; and microcrystalline cellulose, lactose, crospovidone, colloidal silica, and magnesium stearate were sieved through a 40-mesh sieve, respectively.
2) Prescribed amounts of compound A, lactose, and crospovidone were weighed and mixed for 5 min to obtain mixture 1.
3) A prescribed amount of microcrystalline cellulose was added to mixture 1 and mixed for 5 min to obtain mixture 2.
4) Prescribed amounts of colloidal silica and magnesium stearate were added to mixture 2 and mixed for 5 min to obtain an intermediate powder. The intermediate powder was analysed by HPLC, and the content of compound A was found to be 47.5% - 52.5%. HPLC conditions: octadecylsilane-bonded silica gel was used as a packing material; mobile phase: water (adjusted to pH 3.5 with phosphoric acid)/methanol = 38/62; column temperature: 45°C; flow rate: 1 mL/min; detection wavelength: 221 nm.
5) Based on the content of the intermediate powder, tablet compression was carried out using a φ 10 mm die to produce tablets with a strength of 200 mg, where the tablets were controlled to have a weight variation of ± 5% and a hardness of 70-110 N.
6) The prepared tablets were coated with a coating weight gain of about 2.0-5.0%.

### Example 29

| **Component** | **Unit/mg** | **Percentage/%** |
|---|---|---|
| Compound A | 50 | 42.0 |
| Microcrystalline cellulose | 60 | 50.0 |
| Croscarmellose sodium | 8 | 6.6 |
| Colloidal silica | 2 | 1.6 |
| Gelatine hollow capsule | 2# | / |

### Preparation method:

1) Compound A was micronized using a jet pulverization process, with a particle size X90 being controlled to ≤ 40 µm, and sieved through a 60-mesh sieve; and microcrystalline cellulose, croscarmellose sodium, and colloidal silica were sieved through a 60-mesh sieve, respectively.
2) A prescribed amount of compound A was weighed, and a prescribed amount of croscarmellose sodium was added and mixed for 5 min to obtain mixture 1.
3) A prescribed amount of microcrystalline cellulose was added to mixture 1, mixed for 10 min, and sieved through a 60-mesh sieve to obtain mixture 2.
4) A prescribed amount of colloidal silica was added to mixture 2 and mixed for 5 min to obtain a content powder.
5) The capsule was filled with the content powder: the content weight was 120 mg/granule (with a strength of 50 mg).

### Example 30

| **Component** | **Unit/mg** | **Percentage/%** |
|---|---|---|
| Compound A | 100 | 42.0 |
| Microcrystalline cellulose | 120 | 50.0 |
| Croscarmellose sodium | 16 | 6.6 |
| Colloidal silica | 4 | 1.6 |
| Gelatine hollow capsule | 0# | / |

### Preparation method:

1) Compound A was micronized using a jet pulverization process, with a particle size X90 being controlled to ≤ 40 µm, and sieved through a 60-mesh sieve; and microcrystalline cellulose, croscarmellose sodium, and colloidal silica were sieved through a 60-mesh sieve, respectively.
2) A prescribed amount of compound A was weighed, and a prescribed amount of croscarmellose sodium was added and mixed for 5 min to obtain mixture 1.
3) A prescribed amount of microcrystalline cellulose was added to mixture 1, mixed for 10 min, and sieved through a 60-mesh sieve to obtain mixture 2.
4) A prescribed amount of colloidal silica was added to mixture 2 and mixed for 5 min to obtain a content powder.
5) The capsule was filled with the content powder: the content weight was 240 mg/granule (with a strength of 100 mg).

### Dissolution test

### I. Dissolution assay method:

According to the dissolution rate test method (Chinese Pharmacopoeia 2020 Edition Volume IV 0931, Method 2), 900 ml of a hydrochloric acid solution (pH = 1.2) containing 0.1% sodium dodecyl sulphate as a dissolution medium was added to the samples from **Example 1, Example** 2, **Example 3, Example 4, Example 5, Example 6, Example 7, Example 8, Example 9, Example 10, Example 11, Example 12, and Example 13,** respectively; and with a rotation speed of 75 revolutions per minute, sampling was conducted at 5, 10, 15, 20, 30, 45, 60, 90, and 120 minutes to determine the dissolution rate.

### II. Dissolution results and conclusion:

**Table 1 Dissolution rate results**

| Time | Cumulative dissolution rate/% | | | | | | | | |
|---|---|---|---|---|---|---|---|---|---|
| | 5 min | 10 min | 15 min | 20 min | 30 min | 45 min | 60 min | 90 min | 120 min |
| **Example 1** | 23.52 | 35.74 | 44.49 | 51.03 | 60.32 | 69.39 | 75.44 | 83.40 | 87.94 |
| **Example 2** | 17.52 | 27.98 | 45.62 | 55.04 | 67.36 | 78.58 | 87.15 | 92.43 | 95.33 |
| **Example 3** | 22.69 | 41.65 | 51.46 | 58.56 | 67.63 | 75.87 | 80.81 | 86.32 | 89.50 |
| **Example 4** | 15.54 | 28.60 | 36.20 | 41.03 | 48.82 | 56.80 | 62.39 | 70.26 | 75.77 |
| **Example 5** | 43.39 | 57.54 | 66.24 | 71.63 | 78.98 | 84.80 | 87.95 | 91.26 | 92.83 |
| **Example 6** | 40.70 | 53.19 | 61.70 | 66.82 | 74.04 | 80.07 | 83.63 | 87.41 | 89.53 |
| **Example 7** | 32.47 | 46.62 | 56.02 | 62.93 | 72.43 | 81.09 | 86.18 | 92.42 | 95.51 |
| **Example 8** | 30.71 | 44.02 | 52.81 | 59.24 | 68.08 | 76.13 | 80.76 | 86.48 | 89.22 |
| **Example 9** | 40.73 | 59.36 | 68.82 | 74.56 | 80.78 | 85.25 | 87.58 | 90.15 | 91.87 |
| **Example 10** | 29.05 | 42.73 | 52.31 | 58.88 | 67.75 | 75.67 | 80.12 | 85.02 | 88.20 |
| **Example 11** | 36.06 | 51.93 | 61.40 | 67.99 | 76.14 | 82.42 | 85.81 | 89.14 | 90.73 |
| **Example 12** | 21.43 | 40.21 | 55.84 | 64.91 | 74.61 | 81.14 | 84.18 | 87.47 | 89.22 |
| **Example 13** | 46.67 | 64.08 | 72.81 | 78.18 | 84.50 | 88.95 | 91.64 | 94.74 | 95.88 |

Conclusions: The preparations of the present invention had a good drug dissolution effect within 120 min.

### Stability test

### I. Test conditions:

The samples were placed in a clean open watch glass and exposed to conditions of a high-temperature test (60 ± 2°C), a high-humidity test (RH 75% ± 1%), and a light exposure test (4500 ± 500 Lx). Sampling was conducted for testing on 5 days and 10 days, respectively.

### II. Results and conclusion

**Table 2 Test results of influencing factors**

| **High-temperature test (60°C)** | | | | | | |
|---|---|---|---|---|---|---|
| **Investigated item** | **Example 14** | | | **Example 15** | | |
| | **0 day** | **5 days** | **10 days** | **0 day** | **5 days** | **10 days** |
| Characteristic | Off-white after coating removal | Off-white after coating removal | Off-white after coating removal | Off-white after coating removal | Off-white after coating removal | Off-white after coating removal |
| Total amount of impurities | 0.098% | 0.137% | 0.213% | 0.102% | 0.135% | 0.295% |
| Content% | 102.34% | 103.54% | 103.26% | 102.61% | 100.60% | 100.72% |

| **High-humidity test (humidity: 75% ± 1%)** | | | | | | |
|---|---|---|---|---|---|---|
| **Investigated item** | **Example 14** | | | **Example 15** | | |
| | **0 day** | **5 days** | **10 days** | **0 day** | **5 days** | **10 days** |
| Characteristic | Off-white after coating removal | Off-white after coating removal | Off-white after coating removal | Off-white after coating removal | Off-white after coating removal | Off-white after coating removal |
| Total amount of impurities | 0.098% | 0.130% | 0.133% | 0.102% | 0.127% | 0.127% |
| Content% | 102.34% | 103.45% | 103.63% | 102.61% | 101.99% | 100.65% |

| **Light exposure test (illuminance: 4500 lx ± 500 lx)** | | | | | | |
|---|---|---|---|---|---|---|
| **Investigated item** | **Example 14** | | | **Example 15** | | |
| | **0 day** | **5 days** | **10 days** | **0 day** | **5 days** | **10 days** |
| Characteristic | Off-white after coating removal | Off-white after coating removal | Off-white after coating removal | Off-white after coating removal | Off-white after coating removal | Off-white after coating removal |
| Total amount of impurities | 0.098% | 0.129% | 0.136% | 0.102% | 0.132% | 0.144% |
| Content% | 102.34% | 104.33% | 104.10% | 102.61% | 101.43% | 101.77% |

Conclusions: The influencing factor test of the preparations of the present invention showed no notable differences in all tested indicators compared to day 0, indicating that the preparations of the present invention had stable quality.

### Study of pharmacokinetics in beagle dogs

The test substance tablets were prepared for intragastric administration. Healthy male beagle dogs (supplied by the Sichuan Institute of Musk Deer Breeding) were used, and the body weight of the beagle dogs was 10 kg per animal. Each type of tablet was administered to 3 animals at a dose of 1 tablet per animal. At each time point, blood samples were collected from the forelimb vein of all three animals. The test substance was administered via intragastric administration, and blood samples were collected at 15 min, 0.5 h, 1 h, 2 h, 4 h, 8 h, and 24 h after administration. After anticoagulation with EDTA-K2, the blood samples were centrifuged at 3200 g at 4°C for 10 min to separate plasma, and all plasma samples were stored at -70°C for testing. The drug concentration of the test substance in plasma was determined by LC-MS/MS method (with gliclazide as an internal standard), and the main pharmacokinetic parameters were calculated by Winnolin 8.3 non-compartment model.

Conclusions: The preparations of the present invention had good pharmacokinetic characteristics.

The specification of the present invention describes specific embodiments in detail, and those skilled in the art should realize that the above embodiments are exemplary and should not be understood as limiting the present invention. For those skilled in the art, without departing from the principle of the present invention, several improvements and modifications are made to the present invention, and the technical solutions obtained by these improvements and modifications also fall within the scope of protection of the claims of the present invention.

## Claims

1. A pharmaceutical preparation of a piperazine derivative, **characterized by** comprising the piperazine derivative, or a stereoisomer, tautomer, deuterated compound or pharmaceutically acceptable salt thereof, wherein the pharmaceutical preparation comprises the piperazine derivative in a strength of 1-1000 mg, and the piperazine derivative is selected from a compound of formula (I): 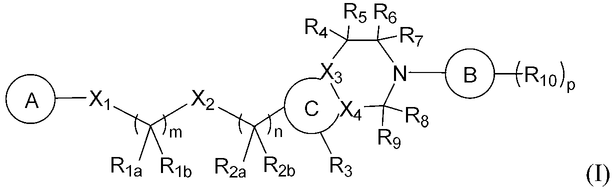 wherein:
X₁ is NH, O or 4- to 6-membered heterocycle containing 1 to 3 heteroatoms selected from N and O;
X₂ is O or a bond;
X₃ and X₄ are each independently C or N;
R₁ₐ and R_{1b} are each independently H, D or C₁₋₆ alkyl; or R₁ₐ and R_{1b} together with the carbon atom connected thereto form 3- to 5-membered cycloalkyl;
R₂ₐ and R_{2b} are each independently H, D or C₁₋₆ alkyl; or R₂ₐ and R_{2b} together with the carbon atom connected thereto form 3- to 5-membered cycloalkyl;
R₃ is H, D, C₁₋₆ alkyl, halogen or cyano, wherein the C₁₋₆ alkyl is optionally substituted with 1 to 3 halogens;
R₄ and R₅ are each independently H, D or C₁₋₆ alkyl; or R₄ and R₅ together with the carbon atom connected thereto form 3- to 5-membered cycloalkyl;
R₆ and R₇ are each independently H, D or C₁₋₆ alkyl; or R₆ and R₇ together with the carbon atom connected thereto form =O;
R₈ and R₉ are each independently H, D or C₁₋₆ alkyl; or R₈ and R₉ together with the carbon atom connected thereto form =O; or R₈ and R₉ together with the carbon atom connected thereto form 3- to 5-membered cycloalkyl;
each R₁₀ is independently C₁₋₆ alkyl, C₁₋₆ alkoxy, CONR₁₀ₐR_{10b}, halogen, cyano, S(O)₂R_{10c}, SR_{10d} or 3- to 5-membered cycloalkyl, wherein the C₁₋₆ alkyl and C₁₋₆ alkoxy are optionally substituted with 1 to 3 halogens;
R₁₀ₐ, R_{10b}, R_{10c} and R_{10d} are each independently H, D or C₁₋₆ alkyl;
A is 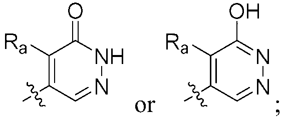
Rₐ is C₁₋₆ alkyl, C₃₋₅ cycloalkyl, halogen or cyano, wherein the C₁₋₆ alkyl is optionally substituted with 1 to 3 halogens;
B is 5- to 10-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3;
n is 0, 1, 2 or 3; and
p is 0, 1, 2 or 3.

2. The pharmaceutical preparation according to claim 1, **characterized in that**
X₁ is NH, O or 4- to 6-membered heterocycle containing 1 to 3 heteroatoms selected from N and O;
X₂ is O or a bond;
X₃ and X₄ are each independently C or N;
R₁ₐ and R_{1b} are each independently H, D or C₁₋₆ alkyl;
R₂ₐ and R_{2b} are each independently H, D or C₁₋₆ alkyl; or R₂ₐ and R_{2b} together with the carbon atom connected thereto form 3- to 5-membered cycloalkyl;
R₃ is H, D, C₁₋₆ alkyl, halogen or cyano, wherein the C₁₋₆ alkyl is optionally substituted with 1 to 3 halogens;
R₄ and R₅ are each independently H, D or C₁₋₆ alkyl; or R₄ and R₅ together with the carbon atom connected thereto form 3- to 5-membered cycloalkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form =O;
each R₁₀ is independently C₁₋₆ alkyl, C₁₋₆ alkoxy, CONR₁₀ₐR_{10b}, halogen, cyano, S(O)₂R_{10c}, SR_{10d} or 3- to 5-membered cycloalkyl, wherein the C₁₋₆ alkyl and C₁₋₆ alkoxy are optionally substituted with 1 to 3 halogens;
R₁₀ₐ, R_{10b}, R_{10c} and R_{10d} are each independently H, D or C₁₋₆ alkyl;
A is 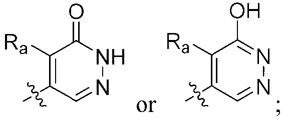
Rₐ is C₁₋₆ alkyl, C₃₋₅ cycloalkyl, halogen or cyano, wherein the C₁₋₆ alkyl is optionally substituted with 1 to 3 halogens;
B is 5- to 6-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3;
n is 0, 1, 2 or 3; and
p is 0, 1, 2 or 3.

3. The pharmaceutical preparation according to claim 1, **characterized in that** the piperazine derivative is selected from a compound of formula (1-1): 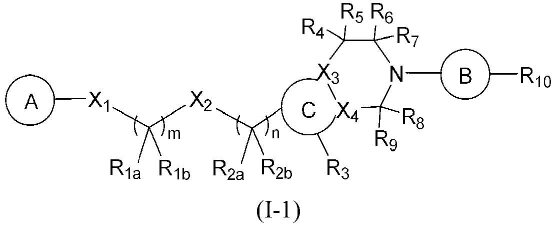 wherein:
X₁ is NH or 4- to 6-membered heterocycle containing 1 to 3 heteroatoms selected from N and O;
X₂ is O;
X₃ and X₄ are each independently C or N;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form =O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 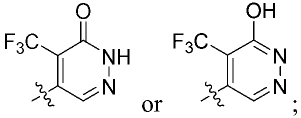
B is 5- to 6-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3; and
n is 0, 1, 2 or 3.

4. The pharmaceutical preparation according to claim 1, **characterized in that** the piperazine derivative is selected from a compound of formula (I-2): 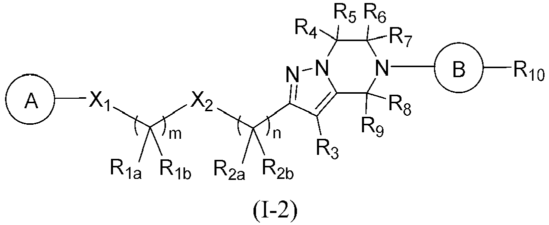 wherein:
X₁ is NH;
X₂ is O;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form =O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 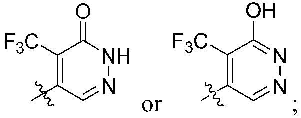
B is 5- to 6-membered carbocycle or heterocycle, wherein the heterocycle contains 1 to 3 heteroatoms selected from N, O and S;
m is 1, 2 or 3; and
n is 0, 1, 2 or 3.

5. The pharmaceutical preparation according to claim 4, **characterized in that**
X₁ is NH;
X₂ is O;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form =O;
R₁₀ is C₁₋₆ alkyl, cyano or SR_{10d}, wherein the C₁₋₆ alkyl is optionally substituted with 1 to 3 halogens;
R_{10d} is H, D or C₁₋₆ alkyl;
A is 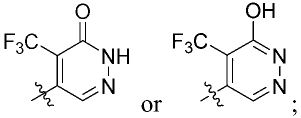
B is 5- to 6-membered carbocycle or heterocycle, wherein the heterocycle contains 1 to 3 heteroatoms selected from N, O and S;
m is 1, 2 or 3; and
n is 0, 1, 2 or 3.

6. The pharmaceutical preparation according to claim 5, **characterized in that**
X₁ is selected from NH;
X₂ is selected from O;
R₁ₐ and R_{1b} are each independently selected from H, D or C₁₋₆ alkyl;
R₂ₐ and R_{2b} are each independently selected from H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H or D;
R₆ and R₇ are each independently H or D;
R₈ and R₉ are each independently H or D;
R₁₀ is CF₃ or SR_{10d};
R_{10d} is H, D or C₁₋₆ alkyl;
A is 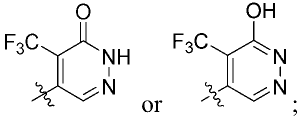
B is 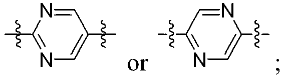
m is 1, 2 or 3; and
n is 0, 1, 2 or 3.

7. The pharmaceutical preparation according to claim 6, **characterized in that**
X₁ is selected from NH;
X₂ is selected from O;
R₁ₐ and R_{1b} are each independently selected from H, D or C₁₋₃ alkyl;
R₂ₐ and R_{2b} are each independently selected from H, D or C₁₋₃ alkyl;
R₃ is selected from H, D or CF₃;
R₄ and R₅ are each independently selected from H or D;
R₆ and R₇ are each independently selected from H or D;
R₈ and R₉ are each independently selected from H or D;
R₁₀ is CF₃;
A is 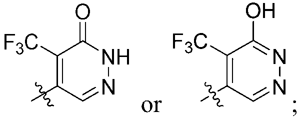
B is 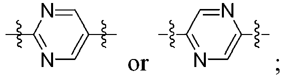
m is 1, 2 or 3; and
n is 0, 1 or 2.

8. The pharmaceutical preparation according to claim 1, **characterized in that** the piperazine derivative is selected from a compound of formula (I-3): 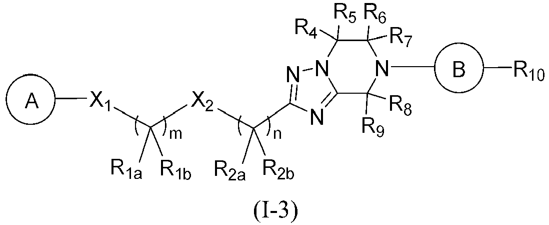 wherein:
X₁ is NH or 4- to 6-membered heterocycle containing 1 to 3 heteroatoms selected from N and O;
X₂ is O;
R₁ₐ and R_{1b} are each independently H, D or C₁₋₆ alkyl;
R₂ₐ and R_{2b} are each independently H, D or C₁₋₆ alkyl;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form =O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 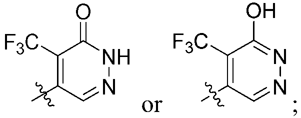
B is 5- to 6-membered carbocycle or heterocycle, wherein the heterocycle contains 1 to 3 heteroatoms selected from N, O and S;
m is 1, 2 or 3; and
n is 0, 1, 2 or 3.

9. The pharmaceutical preparation according to claim 8, **characterized in that**
X₁ is NH or 4- to 6-membered heterocycle containing 1 to 3 heteroatoms selected from N and O;
X₂ is O;
R₁ₐ and R_{1b} are each independently H, D or C₁₋₃ alkyl;
R₂ₐ and R_{2b} are each independently H, D or C₁₋₃ alkyl;
R₄ and R₅ are each independently H, D or C₁₋₃ alkyl;
R₆ and R₇ are each independently H, D or C₁₋₃ alkyl;
R₈ and R₉ are each independently H, D or C₁₋₃ alkyl; or R₈ and R₉ together with the carbon atom connected thereto form =O;
R₁₀ is C₁₋₆ alkyl, cyano or SR_{10d}, wherein the C₁₋₆ alkyl is optionally substituted with 1 to 3 halogens;
R_{10d} is H, D or C₁₋₆ alkyl;
A is 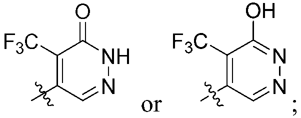
B is 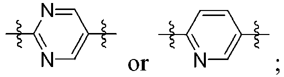
m is 1, 2 or 3; and
n is 0, 1 or 2.

10. The pharmaceutical preparation according to claim 9, **characterized in that**
X₁ is NH;
X₂ is O;
R₁ₐ and R_{1b} are each independently H, D or C₁₋₃ alkyl;
R₂ₐ and R_{2b} are each independently H or D;
R₄ and R₅ are each independently H or D;
R₆ and R₇ are each independently H or D;
R₈ and R₉ are each independently H or D;
R₁₀ is CF₃;
A is 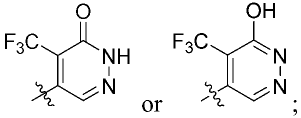
B is 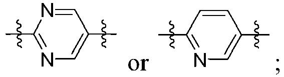
m is 1, 2 or 3; and
n is 0, 1 or 2.

11. The pharmaceutical preparation according to any one of claims 1-10, **characterized in that** the piperazine derivative, or the stereoisomer, tautomer, deuterated compound or pharmaceutically acceptable salt thereof is selected from: 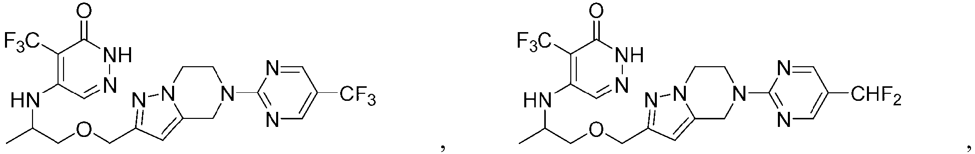 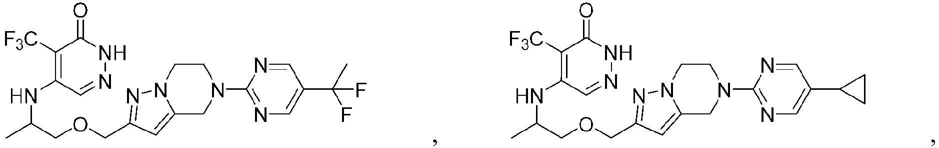 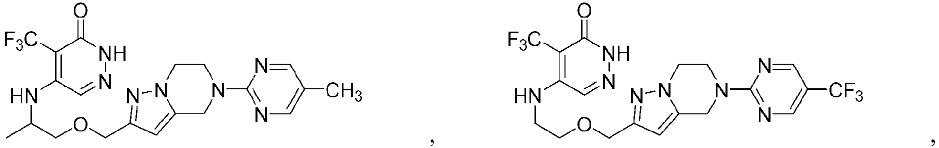 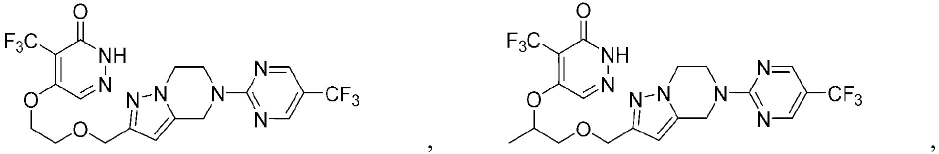 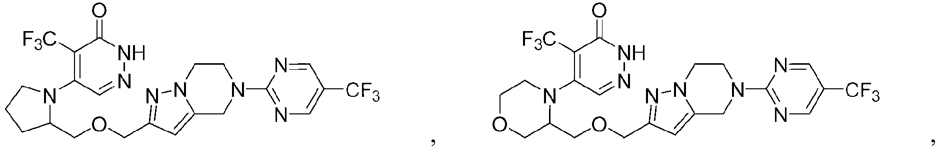 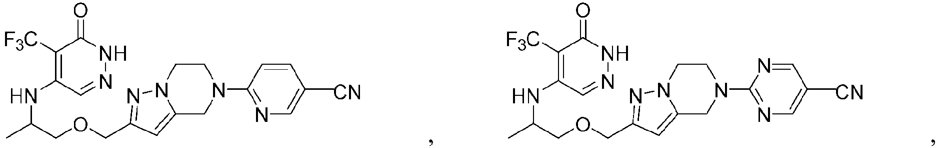 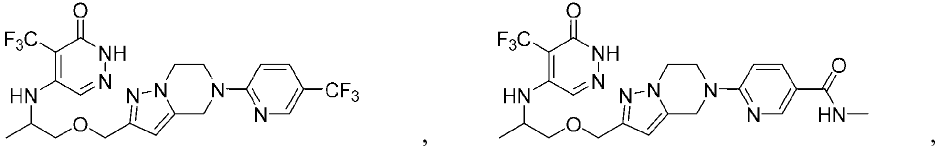 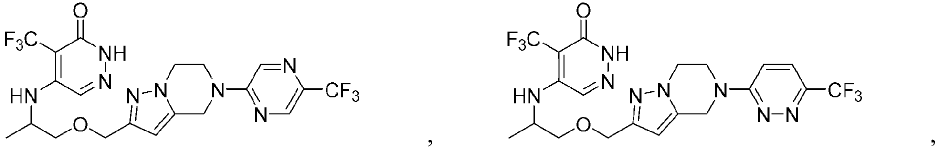 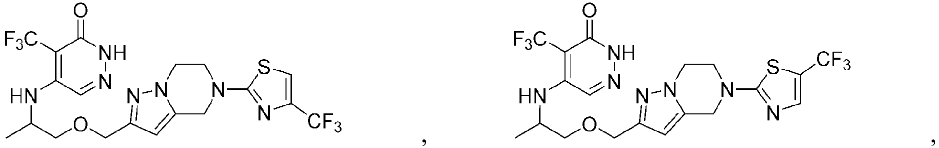 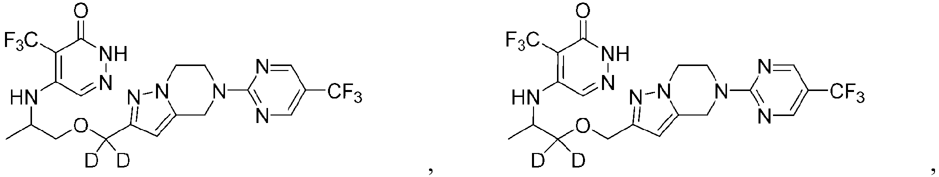 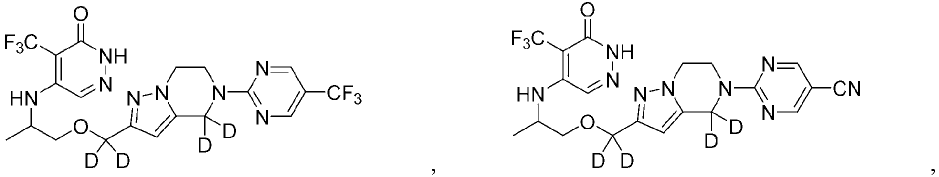 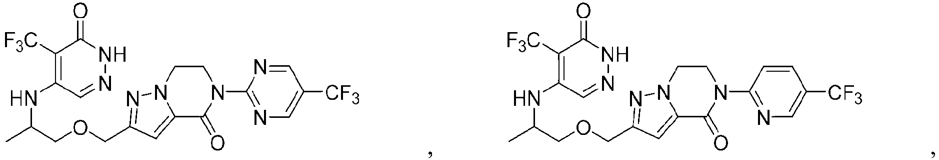 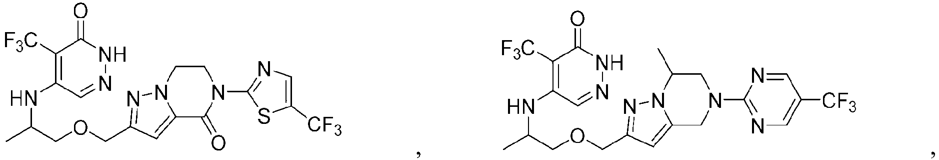 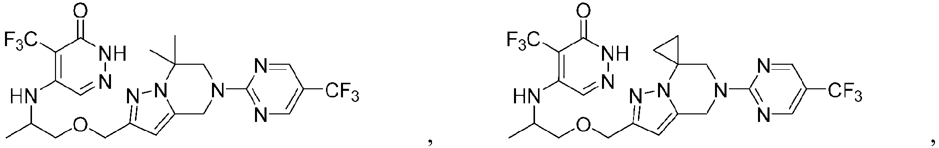 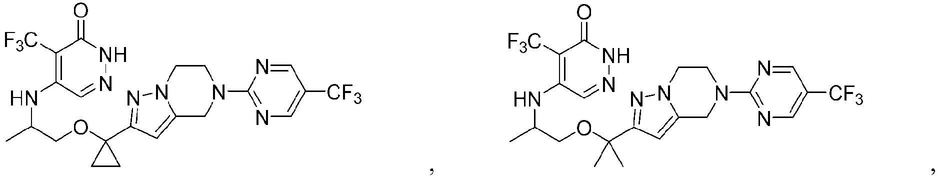 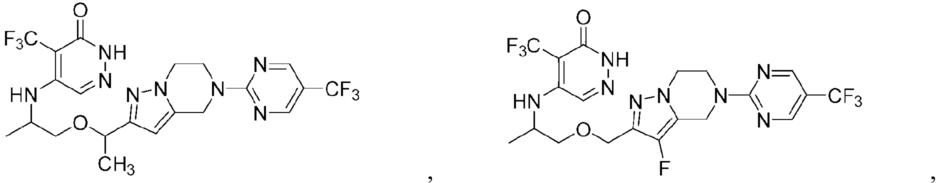 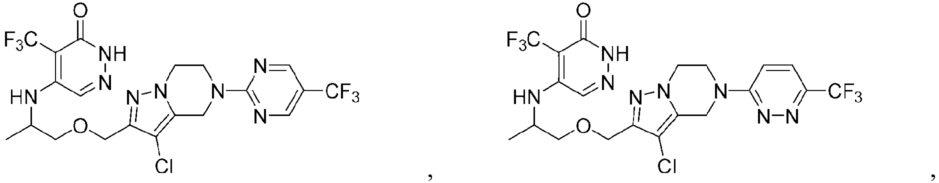 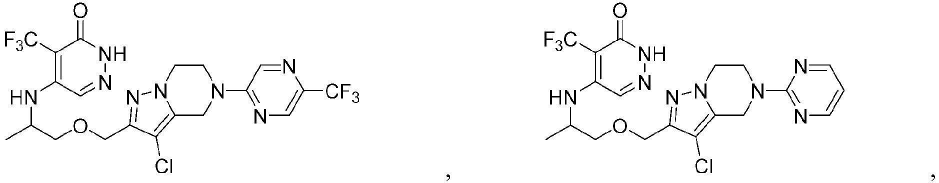 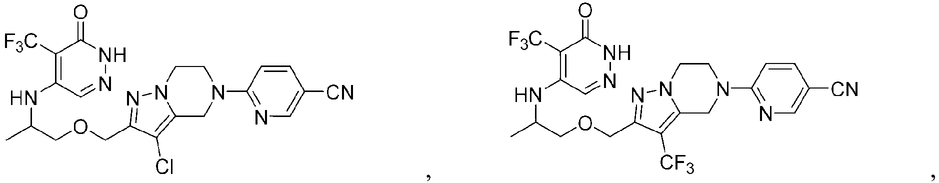 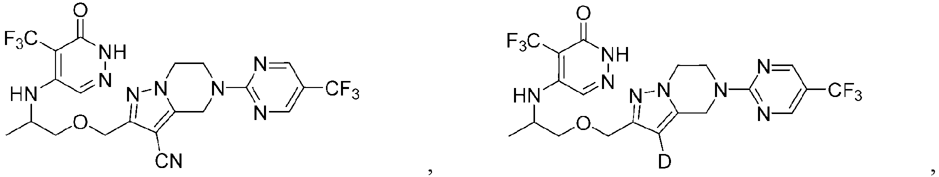 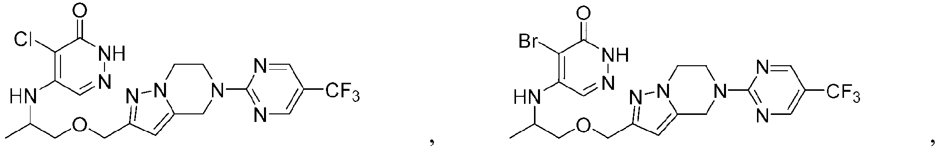 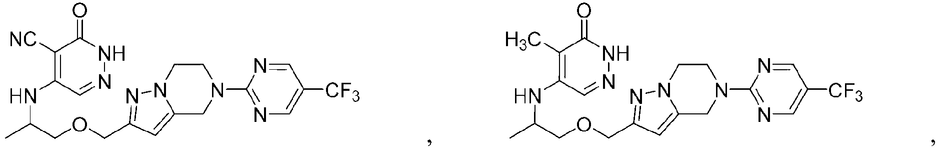 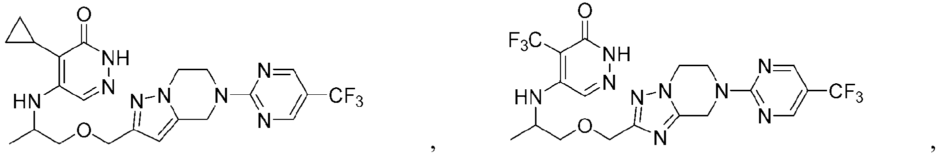 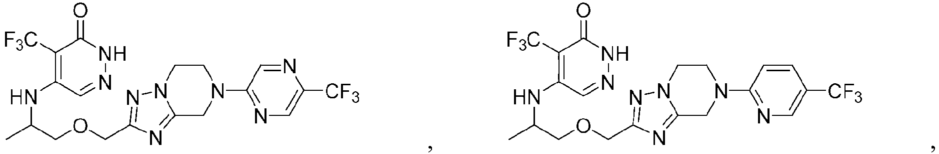 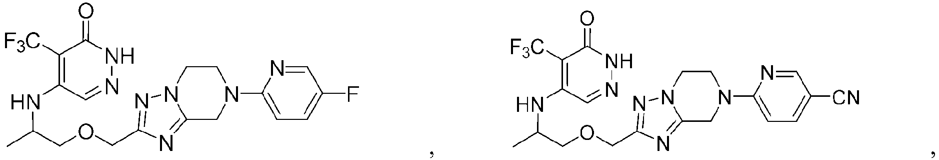 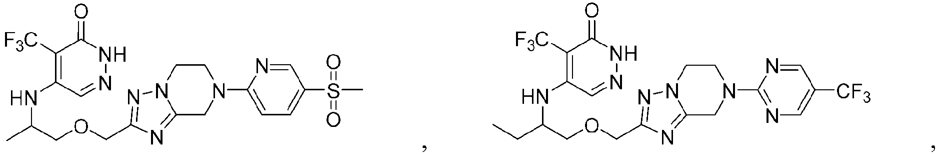 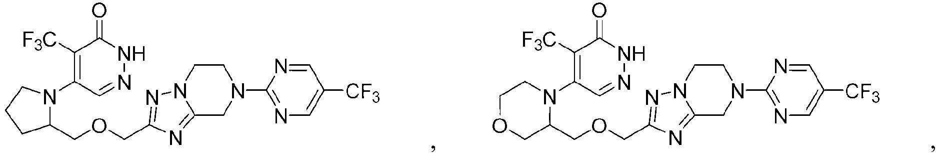 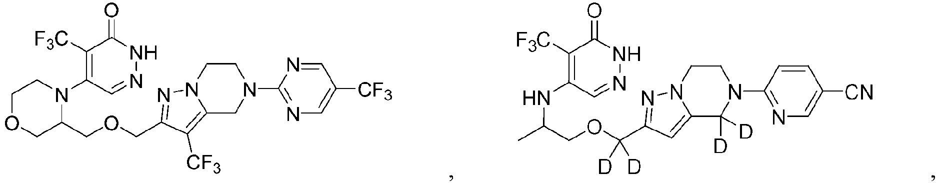 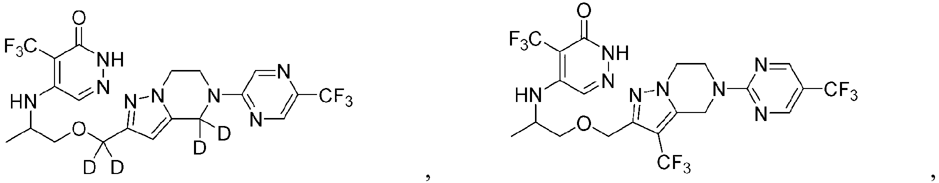 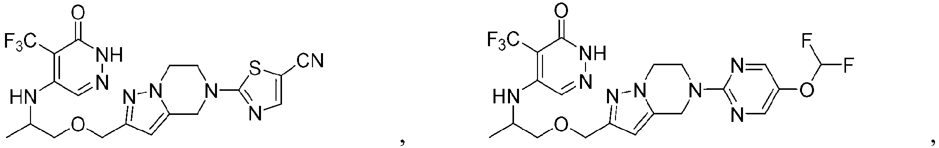 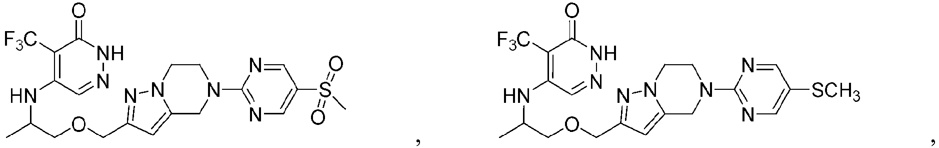 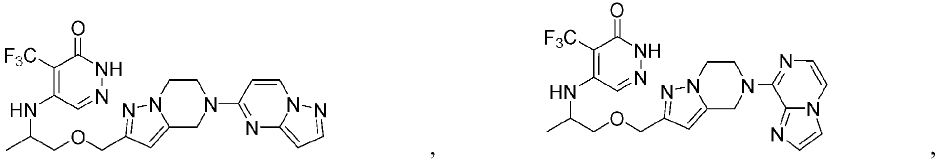 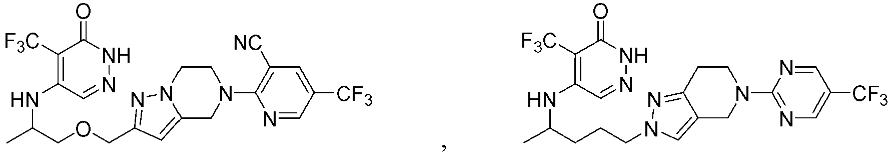 or

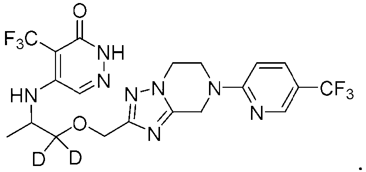

12. The pharmaceutical preparation according to any one of claims 1-11, **characterized in that** the pharmaceutical preparation comprises the piperazine derivative, or the stereoisomer, tautomer, deuterated compound or pharmaceutically acceptable salt thereof in a strength of 1-5 mg, 5-10 mg, 10-15 mg, 15-20 mg, 20-25 mg, 25-30 mg, 30-35 mg, 35-40 mg, 40-50 mg, 50-60 mg, 60-70 mg, 70-80 mg, 80-90 mg, 90-100 mg, 100-150 mg, 150-200 mg, 200-250 mg, 250-300 mg, 300-350 mg, 350-400 mg, 400-450 mg, 450-500 mg, 500-600 mg, 600-700 mg, 700-800 mg, 800-900 mg or 900-1000 mg.

13. The pharmaceutical preparation according to any one of claims 1-12, **characterized in that** the content of the piperazine derivative, or the stereoisomer, tautomer, deuterated compound or pharmaceutically acceptable salt thereof in the pharmaceutical preparation is 5% - 90%, preferably 20% - 80%, and more preferably 30% - 70% of the total weight of the pharmaceutical preparation.

14. The pharmaceutical preparation according to claims 1-13, **characterized in that** the dosage form of the pharmaceutical preparation is selected from a tablet, a capsule, or a granule.

15. The pharmaceutical preparation according to claims 1-14, **characterized in that** the pharmaceutical preparation further comprises one or more of a diluent, a disintegrant, a solubilizer, a glidant, and a lubricant.

16. The pharmaceutical preparation according to claim 15, **characterized in that** the diluent is selected from one or a combination of more of microcrystalline cellulose, starch, mannitol, lactose, pregelatinized starch, dextrin, sucrose, calcium hydrogen phosphate, and calcium phosphate; and the content of the diluent is 10% - 80% of the total weight of the pharmaceutical preparation.

17. The pharmaceutical preparation according to claim 15, **characterized in that** the disintegrant is selected from one or a combination of more of microcrystalline cellulose, croscarmellose sodium, crospovidone, sodium carboxymethyl starch, low-substituted hydroxypropylcellulose, polacrilin potassium, pregelatinized starch, and starch; and the content of the disintegrant is 1% - 60%, and preferably 3% - 20% of the total weight of the pharmaceutical preparation.

18. The pharmaceutical preparation according to claim 15, **characterized in that** the solubilizer is selected from one or a combination of two of poloxamer, and sodium dodecyl sulphate; and the content of the solubilizer is 2% - 10% of the total weight of the pharmaceutical preparation.

19. The pharmaceutical preparation according to claim 15, **characterized in that** the glidant is selected from one or a combination of two of colloidal silica and talc; and the content of the glidant is 0.5% - 5% of the total weight of the pharmaceutical preparation.

20. The pharmaceutical preparation according to claim 15, **characterized in that** the lubricant is selected from one or a combination of more of stearic acid, magnesium stearate, calcium stearate, sodium stearyl fumarate, talc, hydrogenated vegetable oil, magnesium lauryl sulphate, polyethylene glycol, glyceryl behenate, and a sucrose ester; and the content of the lubricant is 0.5% - 5% of the total weight of the pharmaceutical preparation.

21. A preparation method for the pharmaceutical preparation of a piperazine derivative according to any one of claims 1-20, **characterized by** comprising micronizing the piperazine derivative, with the particle size thereof being controlled to 5-100 µm, preferably 5-70 µm, more preferably 5-40 µm; mixing the micronized piperazine derivative with a diluent, a disintegrant and/or a solubilizer to obtain a mixture; subjecting the mixture to dry granulation, and adding a glidant and/or a lubricant to the prepared granules to obtain intermediate granules; then preparing same into a tablet, a capsule, or a granule; and optionally coating the tablet.

22. A preparation method for the pharmaceutical preparation of a piperazine derivative according to any one of claims 1-20, **characterized by** comprising micronizing the piperazine derivative, with the particle size thereof being controlled to 5-100 µm, preferably 5-70 µm, more preferably 5-40 µm; mixing the micronized piperazine derivative with a disintegrant and/or a solubilizer, and then adding a diluent, a glidant and/or a lubricant and mixing same to obtain a mixture; subjecting the mixture to dry granulation, and adding the glidant and/or the lubricant to the prepared granules to obtain intermediate granules; then preparing same into a tablet, a capsule, or a granule; and optionally coating the tablet.

23. A preparation method for the pharmaceutical preparation of a piperazine derivative according to any one of claims 1-20, **characterized by** comprising micronizing the piperazine derivative, with the particle size thereof being controlled to 5-100 µm, preferably 5-70 µm, more preferably 5-40 µm; mixing the micronized piperazine derivative with a diluent and/or a disintegrant, then adding the diluent and mixing same, and then adding a glidant and/or a lubricant and mixing same to obtain a mixture; then preparing same into a tablet, a capsule, or a granule; and optionally coating the tablet.

24. Use of the pharmaceutical preparation of a piperazine derivative according to any one of claims 1-20 in the preparation of a drug for the treatment and/or prevention of a cancer.
